# EUROPÄISCHE PATENTSCHRIFT

(11) **EP 0 818 197 B1**
(45) Veröffentlichungstag und Bekanntmachung des Hinweises auf die Patenterteilung: **12.11.2003**
(21) Anmeldenummer: 97110361.9
(22) Anmeldetag: 25.06.1997
(51) Int. Cl.: A61K 31/435, C07D 401/04, C07D 493/14

(54) **Heterocyclisch kondensierte Pyridine als CETP Inhibitoren**
Heterocyclic condensed pyridines as CETP inhibitors
Pyridines condensés hétérocycliques comme inhibiteurs de CETP

(30) Priorität: 08.07.1996 DE 19627431
(43) Veröffentlichungstag der Anmeldung: 14.01.1998
(73) Patentinhaber: BAYER AG, 51368 Leverkusen (DE)
(72) Erfinder: Schmeck, Carsten, Dr., 42113 Wuppertal (DE); Müller-Gliemann, Matthias, Dr., 42719 Solingen (DE); Schmidt, Gunther, Dr., 42115 Wuppertal (DE); Brandes, Arndt, Dr., 42115 Wuppertal (DE); Angerbauer, Rolf, Dr., Higashinada-ku, Kobe-shi, Hyogo 658 (JP); Lögers, Michael, Dr., 42327 Wuppertal (DE); Bremm, Klaus-Dieter, Dr., 45661 Recklinghausen (DE); Bischoff, Hilmar, Dr., 42113 Wuppertal (DE); Schmidt, Delf, Dr., 42113 Wuppertal (DE); Schuhmacher, Joachim, Dr., 42113 Wuppertal (DE)

(56) Entgegenhaltungen:
- EP-A- 0 325 130
- EP-A- 0 391 185

## Beschreibung

Die vorliegende Erfindung betrifft heterocyclisch kondensierte Pyridine, Verfahren zu ihrer Herstellung und ihre Verwendung in Arzneimitteln.

Aus der US 5 169 857 sind 7-(polysubstituierte Pyridyl)-6-heptenoate zur Behandlung der Arteriosklerose, Lipoproteinaemia und Hyperlipoproteinaemia bekannt. Außerdem wird die Herstellung von 7-(4-Aryl-3-pyridyl)-3,5-dihydroxy-6-heptenoate in der Publikation EP 325 130 A2 beschrieben. Aus der EP 391 185 sind polysubstituierte 2-oxo-1,8-Naphthyridine zur Behandlung der Arteriosklerose, lipoproteinaemia und Hyperlipoproteinaemia bekannt.

Die vorliegende Erfindung betrifft heterocyclisch kondensierte Pyridine der allgemeinen Formel (I), 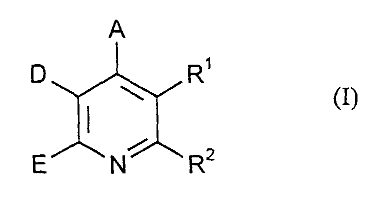 in welcher
- A: für Aryl mit 6 bis 10 Kohlenstoffatomen steht, das gegebenenfalls bis zu 5-fach gleich oder verschieden durch Halogen, Hydroxy, Trifluormethyl, Nitro, Trifluormethoxy oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR³R⁴ substituiert ist,
worin
R³ und R⁴ gleich oder verschieden sind und Wasserstoff, Phenyl oder geradkettiges oder verzweigtes Alkyl mit bis zu 6 Kohlenstoffatomen bedeuten,
- D: für einen Rest der Formel

R⁵―X―

oder 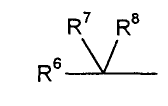 steht,
worin
R⁵ und R⁶ unabhängig voneinander Cycloalkyl mit 3 bis 8 Kohlenstoffatomen bedeuten, oder
Aryl mit 6 bis 10 Kohlenstoffatomen,
oder einen 5- bis 7-gliedrigen aromatischen, gegebenenfalls benzokondensierten Heterocyclus mit bis zu 3 Heteroatomen aus der Reihe S, N und/oder O bedeuten, die gegebenenfalls bis zu 5-fach gleich oder verschieden durch Trifluormethyl, Trifluormethoxy, Nitro, Halogen, Hydroxy, Carboxyl, durch geradkettiges oder verzweigtes Alkyl, Acyl, Alkoxy oder Alkoxycarbonyl mit jeweils bis zu 6 Kohlenstoffatomen oder durch Phenyl, Phenoxy oder Thiophenyl substituiert sind, die ihrerseits durch Halogen, Trifluormethyl oder Trifluormethoxy substituiert sein können,
oder die Cyclen gegebenenfalls durch eine Gruppe der Formel -NR⁹R¹⁰ substituiert sind,
worin
R⁹ und R¹⁰ gleich oder verschieden sind und die oben angegebene Bedeutung von R³ und R⁴ haben,
X eine geradkettige oder verzweigte Alkylen oder Alkenylenkette mit jeweils bis zu 8 Kohlenstoffatomen bedeutet, die gegebenenfalls bis zu 2-fach durch Hydroxy substituiert sind,
R⁷ Wasserstoff oder Halogen bedeutet und
R⁸ Wasserstoff, Halogen, Azido, Trifluormethyl, Hydroxy, Trifluormethoxy, geradkettiges oder verzweigtes Alkoxy mit bis zu 5 Kohlenstoffatomen oder einen Rest der Formel -NR¹¹R¹² bedeutet,
worin
R¹¹ und R¹² gleich oder verschieden sind und die oben angegebene Bedeutung von R³ und R⁴ haben, oder
R⁷ und R⁸ gemeinsam mit dem C-Atom eine Carbonylgruppe bilden,
- E: für Cycloalkyl mit 3 bis 8 Kohlenstoffatomen steht, oder für geradkettiges oder verzweigtes Alkyl mit bis zu 8 Kohlenstoffatomen steht, das gegebenenfalls durch Cycloalkyl mit 3 bis 8 Kohlenstoffatomen oder durch Hydroxy substituiert ist,
- R¹ und R²: gemeinsam eine Alkylenkette mit bis zu 6 Kohlenstoffatomen bilden, die durch ein Sauerstoff- oder Schwefelatom oder durch die Gruppe -SO₂oder -NR¹³ unterbrochen ist,
wobei
R¹³ Wasserstoff oder geradkettiges oder verzweigtes Alkyl oder Acyl mit jeweils bis zu 6 Kohlenstoffatomen bedeutet, oder Benzyl oder Phenyl bedeutet, die gegebenenfalls bis zu 2-fach gleich oder verschieden durch Halogen, Hydroxy, Nitro, Trifluormethyl oder geradkettiges oder verzweigtes Alkyl oder Acyl mit jeweils bis zu 6 Kohlenstoffatomen substituiert sind, und wobei der so gebildete heterocyclische Ring, der auch benzokondensiert sein kann und eine Doppelbindung enthalten kann, stets durch eine Oxogruppe oder einen Rest der Formel 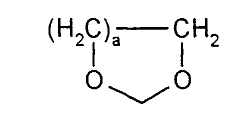 oder -OR¹⁴ substituiert sein muß,
worin
a eine Zahl 1, 2 oder 3 bedeutet und
R¹⁴ Wasserstoff oder geradkettiges oder verzweigtes Alkyl, hydroxysubstituiertes Alkyl, Acyl oder Alkoxycarbonyl mit jeweils bis zu 6 Kohlenstoffatomen oder einen Rest der Formel -SiR¹⁵R¹⁶R¹⁷ bedeutet,
worin
R¹⁵, R¹⁶ und R¹⁷ gleich oder verschieden sind und Phenyl, geradkettiges oder verzweigtes Alkyl mit bis zu 6 Kohlenstoffatomen bedeuten,
und der heterocyclische und/oder benzokondensierte Ring (R¹/R²) gegebenenfalls bis zu 5-fach gleich oder verschieden, gegebenenfalls auch geminal, durch geradkettiges oder verzweigtes Alkoxy oder Alkoxycarbonyl mit jeweils bis zu 6 Kohlenstoffatomen, Trifluormethyl, Halogen, Hydroxy, Carbonyl oder Phenyl substituiert ist, das seinerseits durch Halogen, Trifluormethyl, Nitro, Hydroxy oder durch geradkettiges oder verzweigtes Alkyl, Alkoxy oder Alkoxycarbonyl mit jeweils bis zu 6 Kohlenstoffatomen substituiert sein kann,
und/oder gegebenenfalls bis zu 6-fach, gegebenenfalls auch geminal, gleich oder verschieden durch Cycloalkyl- oder Cycloalkyloxy mit jeweils 3 bis 8 Kohlenstoffatomen oder durch geradkettiges oder verzweigtes Alkyl mit bis zu 6 Kohlenstoffatomen substituiert ist, das seinerseits durch Hydroxy, Trifluormethyl, Phenyl oder durch geradkettiges oder verzweigtes Alkoxy mit bis zu 5 Kohlenstoffatomen substituiert sein kann,
und/oder gegebenenfalls durch einen spiro-verknüpften Rest der Formel 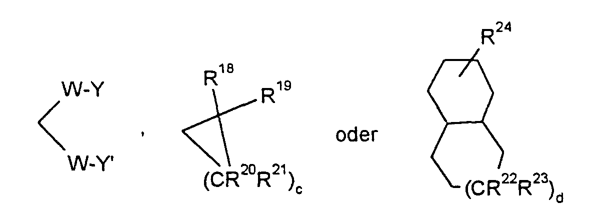 substituiert ist,
worin
- W: entweder ein Sauerstoff oder ein Schwefelatom bedeutet,
- Y und Y': gemeinsam eine 2- bis 6-gliedrige geradkettige oder verzweigte Alkylenkette bilden,
- c: eine Zahl 1, 2, 3, 4, 5, 6 oder 7 bedeutet,
- d: eine Zahl 1 oder 2 bedeutet,
- R¹⁸, R¹⁹, R²⁰, R²¹, R²², R²³ und R²⁴: gleich oder verschieden sind und Wasserstoff, Trifluormethyl, Phenyl, Halogen oder geradkettiges oder verzweigtes Alkyl oder Alkoxy mit jeweils bis zu 6 Kohlenstoffatomen bedeuten oder
- R¹⁸ und R¹⁹ oder R²⁰ und R²¹: jeweils gemeinsam eine geradkettige oder verzweigte Alkylenkette mit bis zu 6 Kohlenstoffatomen bilden oder
- R¹⁸ und R¹⁹ oder R²⁰ und R²¹: jeweils gemeinsam einen Rest der Formel 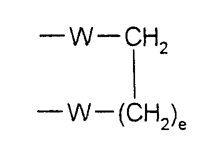 bilden, worin
- W: die oben angegebene Bedeutung hat,
- e: eine Zahl 1, 2, 3, 4, 5 , 6 oder 7 bedeutet,
und deren Salze und N-oxide.

Die erfindungsgemäßen heterocyclisch kondensierten Pyridine können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di-bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Indolyl, Isochinolyl, Chinolyl, Benzo[b]thiophenyl, Benzothiazolyl, Benzo[b]furanyl, Pyridyl, Thienyl, Furyl, Pyrrolyl, Thiazolyl, Oxazolyl, Imidazolyl, Morpholinyl oder Piperidyl. Bevorzugt sind Chinolyl, Indolyl, Pyridyl und Benzothiazolyl.

Bevorzugt sind Verbindungen der allgemeinen Formel (I),
in welcher
- A: für Naphthyl oder Phenyl steht, die gegebenenfalls bis zu 3-fach gleich oder verschieden durch Fluor, Chlor, Brom, Hydroxy, Trifluormethyl, Trifluormethoxy, Nitro oder durch geradkettiges oder verzweigtes Alkyl, Acyl oder Alkoxy mit jeweils bis zu 6 Kohlenstoffatomen oder durch eine Gruppe der Formel -NR³R⁴ substituiert sind,
worin
R³ und R⁴ gleich oder verschieden sind und Wasserstoff, Phenyl oder geradkettiges oder verzweigtes Alkyl mit bis zu 4 Kohlenstoffatomen bedeuten,
- D: für einen Rest der Formel

R⁵―X―

oder 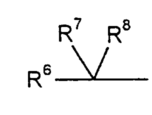 steht,
worin
R⁵ und R⁶ unabhängig voneinander Cyclopropyl, Cyclopentyl oder Cyclohexyl bedeuten, oder Naphthyl, Phenyl, Pyridyl, Chinolyl, Indolyl, Benzthiazolyl oder Tetrahydronaphthalinyl bedeuten, die gegebenenfalls bis zu 3-fach gleich oder verschieden durch Trifluormethyl, Trifluormethoxy, Fluor, Chlor, Brom, Hydroxy, Carboxyl, durch geradkettiges oder verzweigtes Alkyl, Acyl, Alkoxy oder Alkoxycarbonyl mit jeweils bis zu 5 Kohlenstoffatomen oder durch Phenyl, Phenoxy oder Thiophenyl substituiert sind, die ihrerseits durch Fluor, Chlor, Brom, Trifluormethyl oder Trifluormethoxy substituiert sein können,
oder die Cyclen gegebenenfalls durch eine Gruppe der Formel -NR⁹R¹⁰ substituiert sind,
worin
R⁹ und R¹⁰ gleich oder verschieden sind und die oben angegebene Bedeutung von R³ und R⁴ haben,
X eine geradkettige oder verzweigte Alkylen oder Alkenylenkette mit jeweils bis zu 6 Kohlenstoffatomen bedeutet, die gegebenenfalls bis zu 2-fach durch Hydroxy substituiert sind,
R⁷ Wasserstoff, Fluor oder Chlor bedeutet, und
R⁸ Wasserstoff, Fluor, Chlor, Brom, Azido, Trifluormethyl, Hydroxy, Trifluormethoxy, geradkettiges oder verzweigtes Alkoxy mit bis zu 4 Kohlenstoffatomen oder einen Rest der Formel -NR¹¹R¹² bedeutet,
worin
R¹¹ und R¹² gleich oder verschieden sind und die oben angegebene Bedeutung von R³ und R⁴ haben, oder
R⁷ und R⁸ gemeinsam mit dem C-Atom eine Carbonylgruppe bilden,
- E: für Cyclopropyl, Cyclobutyl, Cyclopentyl, Cyclohexyl oder Cycloheptyl steht, oder
für geradkettiges oder verzweigtes Alkyl mit bis zu 6 Kohlenstoffatomen steht, das gegebenenfalls durch Cyclopropyl, Cyclobutyl, Cyclopentyl, Cyclohexyl, Cycloheptyl oder durch Hydroxy substituiert ist,
- R¹ und R²: gemeinsam eine Alkylenkette mit bis zu 5 Kohlenstoffatomen bilden, die durch ein Sauerstoff- oder Schwefelatom oder durch die Gruppe -SO₂oder -NR¹³ unterbrochen ist,
wobei
R¹³ Wasserstoff oder geradkettiges oder verzweigtes Alkyl oder Acyl mit jeweils bis zu 5 Kohlenstoffatomen bedeutet,
oder
Benzyl oder Phenyl bedeutet, die gegebenenfalls bis zu 2-fach gleich oder verschieden durch Fluor, Chlor, Brom, Hydroxy, Nitro, Trifluormethyl oder geradkettiges oder verzweigtes Alkyl oder Acyl mit jeweils bis zu 4 Kohlenstoffatomen substituiert sind, und wobei der so gebildete heterocyclische Ring, der auch benzokondensiert sein kann, und eine Doppelbindung enthalten kann stets durch einen Rest der Formel 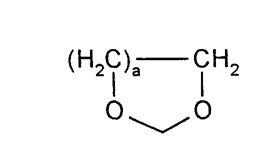 oder -OR¹⁴ substituiert sein muß,
worin
a eine Zahl 1, 2 oder 3 bedeutet und
R¹⁴ Wasserstoff oder geradkettiges oder verzweigtes Alkyl hydroxysubstituiertes Alkyl oder Alkoxycarbonyl mit jeweils bis zu 5 Kohlenstoffatomen oder einen Rest der Formel -SiR¹⁵R¹⁶R¹⁷ bedeutet,
worin
R¹⁵, R¹⁶ und R¹⁷ gleich oder verschieden sind und Phenyl, geradkettiges oder verzweigtes Alkyl mit bis zu 5 Kohlenstoffatomen bedeuten,
und der heterocyclische und/oder benzokondensierte Ring (R¹/R²) gegebenenfalls bis zu 3-fach gleich oder verschieden, gegebenenfalls auch geminal, durch geradkettiges oder verzweigtes Alkoxy oder Alkoxycarbonyl mit jeweils bis zu 4 Kohlenstoffatomen, Hydroxy, Carboxyl oder Phenyl substituiert ist, das seinerseits durch Fluor, Chlor, Brom, Trifluormethyl oder durch geradkettiges oder verzweigtes Alkyl oder Alkoxy mit jeweils bis zu 4 Kohlenstoffatome substituiert sein kann,
und/oder gegebenenfalls bis zu 4-fach, gegebenenfalls auch geminal, gleich oder verschieden durch Cyclopropyl, Cyclopropyloxy, Cyclopentyl, Cyclopentyloxy, Cyclohexyl, Cyclohexyloxy oder durch geradkettiges oder verzweigtes Alkyl mit bis zu 4 Kohlenstoffatomen substituiert ist, das seinerseits durch Hydroxy, Trifluormethyl, Phenyl oder durch geradkettiges oder verzweigtes Alkoxy mit bis zu 3 Kohlenstoffatomen substituiert sein kann
und/oder gegebenenfalls durch einen spiro-verknüpften Rest der Formel 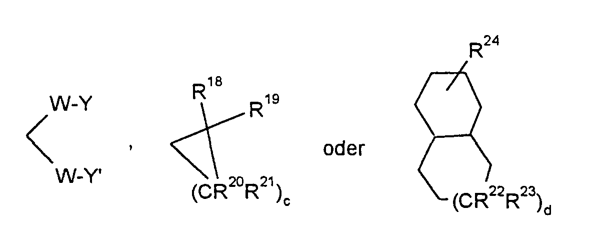 substituiert ist,
worin
- W: entweder ein Sauerstoff oder ein Schwefelatom bedeutet,
- Y und Y': gemeinsam eine 2- bis 5-gliedrige, geradkettige oder verzweigte Alkylenkette bilden,
- c: eine Zahl 1, 2, 3, 4, 5 oder 6 bedeutet,
- d: eine Zahl 1 oder 2 bedeutet,
- R¹⁸, R¹⁹, R²⁰, R²¹, R²², R²³ und R²⁴: gleich oder verschieden sind und Wasserstoff, Trifluormethyl, Phenyl, Fluor, Chlor, Brom oder geradkettiges oder verzweigtes Alkyl oder Alkoxy mit jeweils bis zu 5 Kohlenstoffatomen bedeuten oder
- R¹⁸ und R¹⁹ oder R²⁰ und R²¹: jeweils gemeinsam eine geradkettige oder verzweigte Alkylenkette mit bis zu 5 Kohlenstoffatomen bilden oder
- R¹⁸ und R¹⁹ oder R²⁰ und R²¹: jeweils gemeinsam einen Rest der Formel 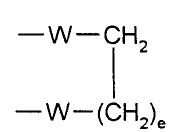 bilden, worin
- W: die oben angegebene Bedeutung hat,
- e: eine Zahl 1, 2, 3, 4, 5 oder 6 bedeutet,
und deren Salze und N-oxide.

Besonders bevorzugt sind die erfindungsgemäßen Verbindungen der allgemeinen Formel (I),
in welcher
- A: für Phenyl steht, das gegebenenfalls durch Fluor, Chlor, Brom, Hydroxy, Trifluormethyl, Trifluormethoxy, Nitro oder durch geradkettiges oder verzweigtes Alkyl oder Alkoxy mit jeweils bis zu 3 Kohlenstoffatomen substituiert ist,
- D: für einen Rest der Formel

R⁵―X―

oder 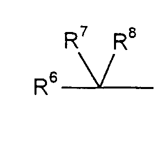 steht,
worin
R⁵ und R⁶ unabhängig voneinander Cyclopropyl, Naphthyl, Phenyl, Pyridyl, Chinolyl, Indolyl, Benzthiazolyl oder Tetrahydronaphthalinyl bedeuten, die gegebenenfalls bis zu 2-fach gleich oder verschieden durch Trifluormethyl, Trifluormethoxy, Fluor, Chlor, Brom, Hydroxy, Carboxyl, Amino, durch geradkettiges oder verzweigtes Alkyl, Acyl, Alkoxy oder Alkoxycarbonyl mit jeweils bis zu 4 Kohlenstoffatomen oder durch Phenyl, Phenoxy oder Thiophenyl substituiert sind, die ihrerseits durch Fluor, Chlor, Brom, Trifluormethyl oder Trifluormethoxy substituiert sein können,
X eine geradkettige oder verzweigte Alkylen- oder Alkenylenkette mit bis zu 4 Kohlenstoffatomen bedeutet, die gegebenenfalls durch Hydroxy substituiert ist,
R⁷ Wasserstoff oder Fluor bedeutet und
R⁸ Wasserstoff, Fluor, Chlor, Brom, Azido, Trifluormethyl, Hydroxy, Amino, Trifluormethoxy oder Methoxy bedeutet, oder
R⁷ und R⁸ gemeinsam mit dem Kohlenstoffatom eine Carbonylgruppe bilden,
- E: für Cyclopropyl, Cyclobutyl, Cyclopentyl, Cyclohexyl oder Cycloheptyl steht, oder
für geradkettiges oder verzweigtes Alkyl mit bis zu 5 Kohlenstoffatomen steht, das gegebenenfalls durch Cyclopentyl oder Cyclohexyl substituiert ist,
- R¹ und R²: gemeinsam eine Alkylenkette mit bis zu 4 Kohlenstoffatomen bilden, die durch ein Sauerstoff- oder Schwefelatom oder durch die Gruppe -SO₂oder -NR¹³ unterbrochen ist,
wobei
R¹³ Wasserstoff, Phenyl oder geradkettiges oder verzweigtes Acyl oder Alkyl jeweils mit bis zu 4 Kohlenstoffatomen bedeutet, oder Benzyl oder Phenyl bedeutet, die gegebenenfalls bis zu 2-fach gleich oder verschieden durch Fluor, Chlor, Brom, Hydroxy, Nitro, Trifluormethyl oder geradkettiges oder verzweigtes Alkyl oder Acyl mit jeweils bis zu 3 Kohlenstoffatomen substituiert sind, und wobei der so gebildete heterocyclische Ring, der auch benzokondensiert sein kann und der eine Doppelbindung enthalten kann, stets durch einen Rest der Formel 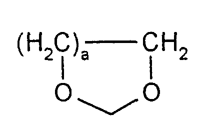 oder -OR¹⁴ substituiert sein muß,
worin
a eine Zahl 1, 2 oder 3 bedeutet und
R¹⁴ Wasserstoff oder geradkettiges oder verzweigtes Alkyl oder hydroxysubstituiertes Alkyl oder Alkoxycarbonyl mit jeweils bis zu 4 Kohlenstoffatomen oder einen Rest der Formel -SiR¹⁵R¹⁶R¹⁷ bedeutet,
worin
R¹⁵, R¹⁶ und R¹⁷ gleich oder verschieden sind und Phenyl, geradkettiges oder verzweigtes Alkyl mit bis zu 4 Kohlenstoffatomen bedeuten,
und der heterocyclische und/oder benzokondensierte Ring (R¹/R²) gegebenenfalls bis zu 3-fach gleich oder verschieden, gegebenenfalls auch geminal, durch geradkettiges oder verzweigtes Alkoxy oder Alkoxycarbonyl mit jeweils bis zu 3 Kohlenstoffatomen, Hydroxy, Carboxyl oder Phenyl substituiert ist, das seinerseits durch Fluor, Chlor, Brom, Trifluormethyl oder durch geradkettiges oder verzweigtes Alkyl mit bis zu 3 Kohlenstoffatomen substituiert sein kann,
und/oder gegebenenfalls bis zu 3-fach, gegebenenfalls auch geminal, gleich oder verschieden durch Cyclopropyl, Cyclopropyloxy, Cyclopentyl, Cyclopentyloxy, Cyclohexyl oder durch geradkettiges oder verzweigtes Alkyl mit bis zu 3 Kohlenstoffatomen substituiert ist, das seinerseits durch Hydroxy, Trifluormethyl, Phenyl oder Methoxy substituiert sein kann
und/oder gegebenenfalls durch einen spiro-verknüpften Rest der Formel 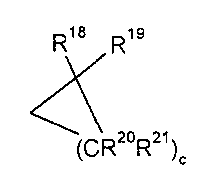 substituiert ist,
worin
- c: eine Zahl 1, 2, 3, 4 oder 5 bedeutet,
R¹⁸, R¹⁹, R²⁰ und R²¹ gleich oder verschieden sind und Wasserstoff, Trifluormethyl, Phenyl, Fluor, Chlor, Brom oder geradkettiges oder verzweigtes Alkyl oder Alkoxy mit jeweils bis zu 4 Kohlenstoffatomen bedeuten
oder
R¹⁸ und R¹⁹ oder R²⁰ und R²¹ jeweils gemeinsam eine geradkettige oder verzweigte Alkylenkette mit bis zu 4 Kohlenstoffatomen bilden
oder
R¹⁸ und R¹⁹ oder R²⁰ und R²¹ jeweils gemeinsam einen Rest der Formel 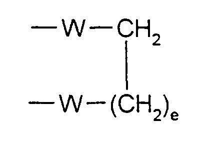 bilden,
worin
- W: entweder ein Sauerstoff- oder Schwefelatom bedeutet und
- e: eine Zahl 1, 2, 3, 4 oder 5 bedeutet,
und deren Salze und N-Oxide.

Ganz besonders bevorzugt sind Verbindungen der allgemeinen Formel (I),
in welcher
- A: für Phenyl steht, das gegebenenfalls durch Fluor substituiert ist
und
- E: für Cyclopentyl oder iso-Propyl steht und deren Salze und N-Oxide.

Außerdem wurde ein Verfahren zur Herstellung der erfindungsgemäßen Verbindungen der allgemeinen Formel (I) gefunden, dadurch gekennzeichnet, daß man in die

Verbindungen der allgemeinen Formel (II) 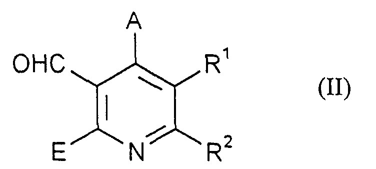 in welcher
- A, E, R¹ und R²: die oben angegebene Bedeutung haben,
zunächst mit metallorganischen Reagenzien im Sinne einer Grignard- oder Wittig-Reaktion den Substituenten D in inerten Lösemitteln einführt,
und gegebenenfalls die unter A, E und/oder R¹ und R² aufgeführten Substituenten nach üblichen Methoden, variiert oder einführt.

Das erfindungsgemäße Verfahren kann durch folgendes Formelschema beispielhaft erläutert werden:

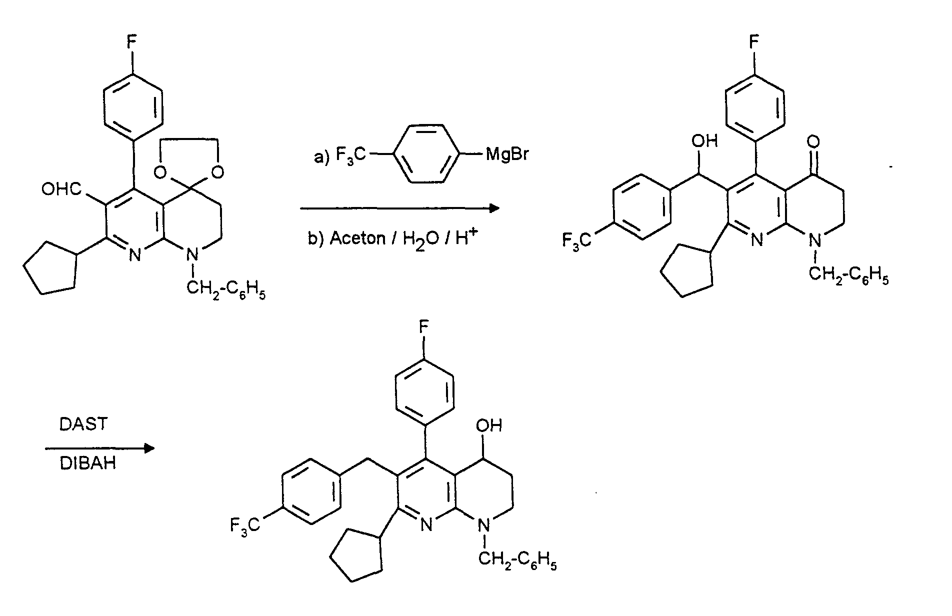

Als Lösemittel eignen sich Ether wie Diethylether, Dioxan, Tetrahydrofuran, Glykoldimethylether, oder Kohlenwasserstoffe wie Benzol, Toluol, Xylol, Hexan, Cylcohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dichlormethan und Tetrahydrofuran.

Als Basen kommen für die einzelnen Schritte die üblichen stark basischen Verbindungen in Frage. Hierzu gehören bevorzugt lithiumorganische Verbindungen wie beispielsweise Lithium-bis-(triethylbutyl)amid, n-Butyllithium, sec. Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid oder Alkoholate wie beispielsweise Kalium-tert.butylat. Besonders bevorzugt wird n-Butyllithium, Natriumhydrid oder Kalium-tert.butylat eingesetzt.

Als metallorganische Reagenzien eignen sich beispielsweise Systeme wie Mg/Brombenzotrifluorid und p-Trifluormethylphenyllithium. Bevorzugt ist das System Mg/Brombenzotrifluorid.

Als Wittig-Reagenzien eignen sich die üblichen Reagenzien. Bevorzugt ist 3-Trifluormethylbenzyltriphenylphosphoniumbromid.

Als Basen eignen sich im allgemeinen eine der oben aufgeführten Basen, vorzugsweise Li-bis-(triethylbutyl)amid oder n-Butyllithium.

Die Base wird in einer Menge von 0,1 mol bis 5 mol, bevorzugt von 0,5 mol bis 2 mol jeweils bezogen auf 1 mol der Ausgangsverbindung eingesetzt.

Die Umsetzung mit Wittig-Reagenzien wird im allgemeinen in einem Temperaturbereich von 0°C bis 150°C, bevorzugt bei 25°C bis 40°C, durchgeführt.

Die Wittig-Reaktionen werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die Reduktionen werden im allgemeinen mit Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat oder Lithiumaluminiumhydrid oder Diisobutylaluminiumhydrid (DIBAH) durchgeführt. Ganz besonders bevorzugt wird die Reduktion mit Natriumborhydrid oder DIBAH, in Anwesenheit von Triethylboran durchgeführt.

Das Reduktionsmittel wird im allgemeinen in einer Menge von 4 mol bis 10 mol, bevorzugt von 4 mol bis 5 mol bezogen auf 1 mol der zu reduzierenden Verbindungen eingesetzt.

Die Reduktion verläuft im allgemeinen in einem Temperaturbereich von -78°C bis +50°C, bevorzugt von -78°C bis 0°C, besonders bevorzugt bei -78°C, jeweils in Abhängigkeit von der Wahl des Reduktionsmittels sowie Lösemittels.

Die Reduktion verläuft im allgemeinen bei Normaldruck, es ist aber auch möglich bei erhöhtem oder erniedrigtem Druck zu arbeiten.

Die Hydrierung erfolgt nach üblichen Methoden mit Wasserstoff in Anwesenheit von Edelmetallkatalysatoren, wie beispielsweise Pd/C, Pt/C oder Raney-Nickel in einem der oben aufgeführten Lösemittel, vorzugsweise in Alkoholen wie beispielsweise Methanol, Ethanol oder Propanol, in einem Temperaturbereich von -20°C bis +100°C, bevorzugt von 0°C bis +50°C, bei Normaldruck oder Überdruck.

Als Derivatisierungen seien beispielhaft folgende Reaktionstypen genannt: Oxidationen, Reduktionen, Hydrierungen, Halogenierung, Wittig/Grignard-Reaktionen und Amidierungen/Sulfoamidierungen.

Als Basen kommen für die einzelnen Schritte die üblichen stark basischen Verbindungen in Frage. Hierzu gehören bevorzugt lithiumorganische Verbindungen wie beispielsweise n-Butyllithium, sec.-Butyllithium, tert.-Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird n-Butyllithium oder Natriumhydrid eingesetzt.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die einzelnen Reaktionsschritte auch Alkohole wie Methanol, Ethanol, Propanol, Butanol oder tert.Butanol. Bevorzugt ist tert.Butanol.

Gegebenenfalls ist es nötig, einige Reaktionsschritte unter Schutzgasatmosphäre durchzuführen.

Die Halogenierungen erfolgen im allgemeinen in einem der oben aufgeführten chlorierten Kohlenwasserstoffen, wobei Methylenchlorid bevorzugt ist.

Als Halogenierungsmittel eignen sich beispielsweise Diethylamino-Schwefeltrifluorid (DAST) oder SOCl₂.

Die Halogenierung verläuft im allgemeinen in einem Temperaturbereich von -78°C. bis +50°C, bevorzugt von -78°C bis 0°C, besonders bevorzugt bei -78°C, jeweils in Abhängigkeit von der Wahl des Halogenierungsmittels sowie Lösemittel.

Die Halogenierung verläuft im allgemeinen bei Normaldurck, es ist aber auch möglich bei erhöhtem oder erniedrigtem Druck zu arbeiten.

Die Verbindungen der allgemeinen Formel (II) sind neu und können hergestellt werden, indem man
[A] zunächst Verbindungen der allgemeinen Formel (III) 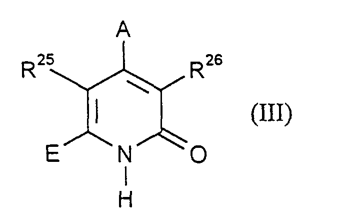 in welcher
   - A und E: die oben angegebene Bedeutung haben,
   und
   - R²⁵ und R²⁶: gleich oder verschieden sind und C₁-C₄-Alkoxycarbonyl bedeuten,
   zunächst mit Phosphoroxychlorid in die Verbindungen der allgemeinen Formel (IV) 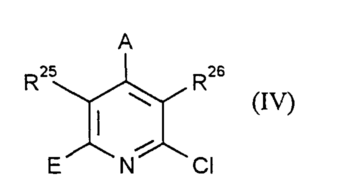 in welcher
   - A, E, R²⁵ und R²⁶: die oben angegebene Bedeutung haben,
   überführt, anschließend in Abhängigkeit der oben angegebenen Bedeutung von R¹ und R² durch eine nucleophile Substitution in inerten Lösemitteln, gegebenenfalls in Anwesenheit einer Base
   in die Verbindungen der allgemeinen Formel (V) 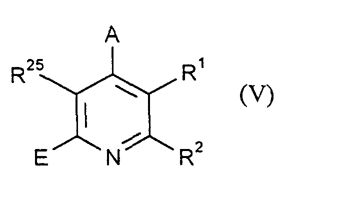 in welcher
   - A, E, R¹, R² und R²⁵: die oben angegebene Bedeutung haben,
   in inerten Lösemitteln und gegebenenfalls in Anwesenheit einer Base, überführt, oder indem man
[B] Verbindungen der allgemeinen Formel (VI) 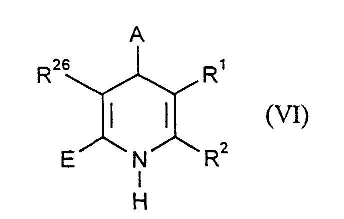 in welcher
   - A, E, R¹ und R²: die oben angegebene Bedeutung haben
   und
   - R²⁶: die oben angegebene Bedeutung hat,
   zunächst durch eine Oxidation in die Verbindungen der allgemeinen Formel (Va) 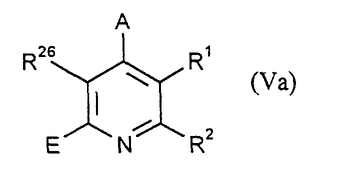 in welcher
   - A, E, R¹, R² und R²⁶: die oben angegebene Bedeutung haben,
   überführt und anschließend wie unter [A] beschrieben weiter umsetzt,
und gegebenenfalls auf allen Stufen Derivatisierungen wie beispielsweise eine Alkylierung oder Halogenierung oder Abspaltungen, oder Einführungen von Schutzgruppen nach üblichen Methoden durchführt,
und in einem letzten Schritt die Alkoxycarbonylfunktion (R²⁶) nach üblichen Methoden zum Aldehyd umsetzt.

Das erfindungsgemäße Verfahren kann durch folgendes Formelschema beispielhaft erläutert werden:

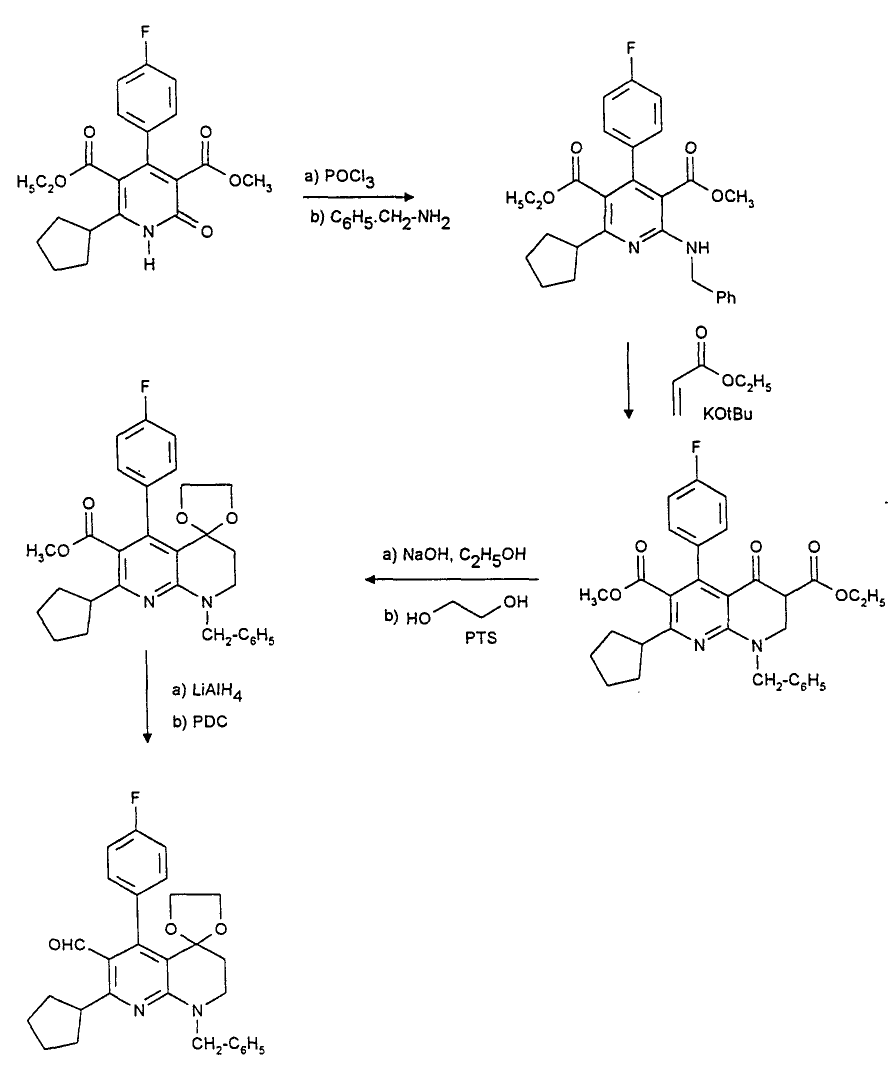

Als Lösemittel eignen sich für die einzelnen Schritte Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan oder Alkohole, wie beispielsweise Methanol, Ethanol oder Propanol. Ebenso ist es möglich, Gemische der genannten Lösemittel zu verwenden. Bevorzugt sind Dichlormethan, Acetonitril, Ethanol und Tetrahydrofuran.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat, Natriummethanolat oder Kalium-tert.butylat oder Natriumhydrogencarbonat. Besonders bevorzugt wird Natriumhydroxid, Kaliumhydroxid oder Kalium-tert.butylat eingesetzt.

Die Base wird im allgemeinen in einer Menge von 0,5 mol bis 5 mol, bevorzugt von 1 mol bis 2,5 mol jeweils bezogen auf 1 mol der Verbindungen der allgemeinen Formeln (V) und (Va) eingesetzt.

Die Umsetzung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die Verbindungen der allgemeinen Formel (III) sind teilweise bekannt oder neu und können dann aber aus den entsprechenden 2-Oxo-1,2,3,4-tetrahydropyridinen durch eine Oxidation hergestellt werden.

Als Lösemittel eignen sich für die Oxidation Ether wie Diethylether, Dioxan, Tetrahydrofuran, Glykoldimethylether, oder Kohlenwasserstoffe wie Benzol, Toluol, Xylol, Hexan, Cylcohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton, Nitromethan oder Wasser. Ebenso ist es möglich, Gemische der genannten Lösemittel zu verwenden. Bevorzugt sind Acetonitril und Wasser.

Als Oxidationsmittel eignen sich beispielsweise Cer(IV)-ammoniumnitrat, 2,3-Dichlor-5,6-dicyan-benzochinon, Pyridiniumchlorochromat (PCC), Osmiumtetroxid und Mangandioxid. Bevorzugt ist Cer(IV)-ammoniumnitrat.

Das Oxidationsmittel wird in einer Menge von 1 mol bis 10 mol, bevorzugt von 2 mol bis 5 mol bezogen auf 1 mol der Verbindungen der allgemeinen Formel (IV) eingesetzt.

Die Oxidation verläuft im allgemeinen in einem Temperaturbereich von -50°C bis +100°C, bevorzugt von 0°C bis Raumtemperatur.

Die Oxidation verläuft im allgemeinen bei Normaldruck. Es ist aber auch möglich, die Oxidation bei erhöhtem oder erniedrigtem Druck durchzuführen.

Die Verbindungen der allgemeinen Formeln (IV) sind größtenteils neu und können wie oben beschrieben hergestellt werden.

Die Verbindungen der allgemeinen Formel (V) und (Va) sind größtenteils neu und können wie oben beschrieben hergestellt werden.

Die Verbindungen der allgemeinen Formel (VI) sind größtenteils neu und können beispielsweise hergestellt werden, indem man

Aldehyde der allgemeinen Formel (VII)

A-CHO (VII)

in welcher
- A: die oben angegebene Bedeutung hat,
mit Verbindungen der allgemeinen Formel (VIII) 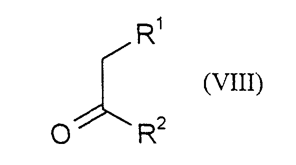 in welcher
- R¹ und R²: die oben angegebene Bedeutung haben,
und der Verbindung der allgemeinen Formel (IX) 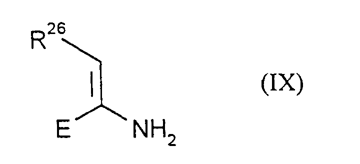 in welcher
- E und R²⁶: die oben angegebene Bedeutung haben,
umsetzt,
in einem der oben aufgeführten Lösemittel, vorzugsweise Ethanol bei Rückflußtemperatur und Normaldruck umsetzt.

Die Verbindungen der allgemeinen Formeln (VII), (VIII) und (IX) sind an sich bekannt oder nach üblichen Methoden herstellbar.

Die erfindungsgemäßen Verbindungen der allgemeinen Formel (I) haben ein nicht vorhersehbares pharmakologisches Wirkspektrum.

Die erfindungsgemäßen Verbindungen der allgemeinen Formel (I) besitzen wertvolle, im Vergleich zum Stand der Technik überlegene, pharmakologische Eigenschaften, insbesondere sind sie hochwirksame Inhibitoren des Cholesterin-Ester-Transfer-Proteins (CETP) und stimulieren den Reversen Cholesterintransport. Die erfindungsgemäßen Wirkstoffe bewirken eine Senkung des LDL-Cholesterinspiegels im Blut bei gleichzeitiger Erhöhung des HDL-Cholesterinspiegels. Sie können deshalb zur Behandlung und Prävention von Hyperlipoproteinämie, Dyslipidämien, Hypertriglyceridämien, Hyperlipidämien oder Arteriosklerose eingesetzt werden.

Die pharmakologischen Wirkungen der erfindungsgemäßen Stoffe wurden in folgendem Test bestimmt:

### CETP-Inhibitions-Testung

### Gewinnung von CETP

CETP wird aus humanem Plasma durch Differential-Zentrifugation und Säulenchromatographie in partiell gereinigter Form gewonnen und zum Test verwendet. Dazu wird humanes Plasma mit NaBr auf eine Dichte von 1,21 g pro ml eingestellt und 18 h bei 50.000 Upm bei 4°C zentrifugiert. Die Bodenfraktion (d>1,21 g/ml) wird auf eine Sephadex®Phenyl-Sepharose 4B (Fa. Pharmacia) Säule aufgetragen, mit 0,15 m NaCl/0,001 m TrisHCl pH 7,4 gewaschen und anschließend mit dest. Wasser eluiert. Die CETP-aktiven Fraktionen werden gepoolt, gegen 50mM NaAcetat pH 4,5 dialysiert und auf eine CM-Sepharose® (Fa. Pharmacia)-Säule aufgetragen. Mit einem linearen Gradienten (0-1 M NaCl) wird anschließend eluiert. Die gepoolten CETP-Fraktionen werden gegen 10 mM TrisHCl pH 7,4 dialysiert und anschließend durch Chromatographie über eine Mono Q®-Säule (Fa. Pharmacia) weiter gereinigt.

### Gewinnung von radioaktiv markiertem HDL

50 ml frisches humanes EDTA-Plasma wird mit NaBr auf eine Dichte von 1,12 eingestellt und bei 4°C im Ty 65-Rotor 18 h bei 50.000 Upm zentrifugiert. Die Oberphase wird zur Gewinnung von kaltem LDL verwendet. Die Unterphase wird gegen 3*4 l PDB-Puffer (10 mM Tris/HCl pH 7,4, 0,15 mM NaCl, 1 mM EDTA, 0,02% NaN₃) dialysiert. Pro 10 ml Retentatvolumen wird anschließend 20 µl ³H-Cholesterin (Dupont NET-725; 1 -µC/µl gelöst in Ethanol) hinzugesetzt und 72 h bei 37°C unter N₂ inkubiert.

Der Ansatz wird dann mit NaBr auf die Dichte 1,21 eingestellt und im Ty 65-Rotor 18 h bei 50.000 Upm bei 20°C zentrifugiert. Man gewinnt die Oberphase und reinigt die Lipoproteinfraktionen durch Gradientenzentrifugation. Dazu wird die isolierte, markierte Lipoproteinfraktion mit NaBr auf eine Dichte von 1,26 eingestellt. Je 4 ml dieser Lösung werden in Zentrifugenröhrchen (SW 40-Rotor) mit 4 ml einer Lösung der Dichte 1,21 sowie 4,5 ml einer Lösung von 1,063 überschichtet (Dichtelösungen aus PDB-Puffer und NaBr) und anschließend 24 h bei 38.000 Upm und 20°C im SW 40-Rotor zentrifugiert. Die zwischen der Dichte 1,063 und 1,21 liegende, das markierte HDL enthaltende Zwischenschicht wird gegen 3*100 Volumen PDB-Puffer bei 4°C dialysiert. Das Retentat enthält radioaktiv markiertes ³H-CE-HDL, das auf ca. 5x10⁶ cpm pro ml eingestellt zum Test verwendet wird.

### CETP-Test

Zur Testung der CETP-Aktivität wird die Übertragung von ³H-Cholesterolester von humanen HD-Lipoproteinen auf biotinylierte LD-Lipoproteine gemessen.
Die Reaktion wird durch Zugabe von Streptavidin-SPA®beads (Fa. Amersham) beendet und die übertragene Radioaktivität direkt im Liquid. Scintillation Counter bestimmt.
Im Testansatz werden 10 µl HDL-³H-Cholesterolester (∼ 50.000 cpm) mit 10 µl Biotin-LDL (Fa. Amersham) in 50 mM Hepes / 0,15 m NaCl / 0,1% Rinderserumalbumin / 0,05% NaN₃ pH 7,4 mit 10 µl CETP (1 mg/ml) und 3 µl Lösung der zu prüfenden Substanz (in 10% DMSO / 1% RSA) gelöst, für 18 h bei 37°C inkubiert. Anschließend werden 200 µl der SPA-Streptavidin-Bead-Lösung (TRKQ 7005) zugesetzt, 1 h unter Schütteln weiter inkubiert und anschließend im Scintillationszähler gemessen. Als Kontrollen dienen entsprechende Inkubationen mit 10 µl Puffer, 10 µl CETP bei 4°C sowie 10 µl CETP bei 37°C.
Die in den Kontrollansätzen mit CETP bei 37°C übertragene Aktivität wird als 100% Übertragung gewertet. Die Substanzkonzentration, bei der diese Übertragung auf die Hälfte reduziert ist, wird als IC₅₀-Wert angegeben.

In der folgenden Tabelle A sind die IC₅₀-Werte (mol/l) für CETP-Inhibitoren angegeben:

**Tabelle A**

| **Beispiel-Nr.** | **IC**_{**50**}**-Wert (mol/l)** |
|---|---|
| 12 | 2 x 10⁻⁷ |
| 14 | 6 x 10⁻⁶ |
| 16 | 8,5 x 10⁻⁷ |

### Ex vivo Aktivität der erfindungsgemäßen Verbindungen

Syrische Goldhamster aus werkseigener Zucht werden nach 24-stündigem Fasten narkotisiert (0,8 mg/kg Atropin, 0,8 mg/kg Ketavet® s.c., 30' später 50 mg/kg Nembutal i.p.). Anschließend wird die V.jugularis freipräpariert und kanüliert. Die Testsubstanz wird in einem geeigneten Lösemittel (in der Regel Adalat-Placebolösung: 60 g Glycerin, 100 ml H₂O, ad 1000 ml PEG-400) gelöst und den Tieren über einen in die V.jugularis eingeführten PE-Katheter verabreicht. Die Kontrolltiere erhalten das gleiche Volumen Lösungsmittel ohne Testsubstanz. Anschließend wird die Vene abgebunden und die Wunde verschlossen.
Die Verabreichung der Testsubstanzen kann auch p.o. erfolgen, indem die Substanzen in DMSO gelöst und 0,5% Tylose suspendiert mittels. Schlundsonde peroral verabreicht werden. Die Kontrolltiere erhalten identische Volumen Lösemittel ohne Testsubstanz.
Nach verschiedenen Zeitpunkten - bis zu 24 Stunden nach Applikation - wird den Tieren durch Punktion des retro-orbitalen Venenplexus Blut entnommen (ca. 250 µl). Durch Inkubation bei 4°C über Nacht wird die Gerinnung abgeschlossen, anschließend wird 10 Minuten bei 6000 x g zentrifugiert. Im so erhaltenen Serum wird die CETP-Aktivität durch den modifizierten CETP-Test bestimmt. Es wird wie für den CETP-Test oben beschrieben die Übertragung von ³H-Cholesterolester von HD-Lipoproteinen auf biotinylierte LD-Lipoproteine gemessen.
Die Reaktion wird durch Zugabe von Streptavidin-SPA^{R}beads (Fa. Amersham) beendet und die übertragene Radioaktivität direkt im Liquid Scintlation Counter bestimmt.
Der Testansatz wird wie unter "CETP-Test" beschrieben durchgeführt. Lediglich 10 µl CETP werden für die Testung der Serum durch 10 µl der entsprechenden Serumproben ersetzt. Als Kontrollen dienen entsprechende Inkubationen mit Seren von unbehandelten Tieren.
Die in den Kontrollansätzen mit Kontrollseren übertragene Aktivität wird als 100% Übertragung gewertet. Die Substanzkonzentration, bei der diese Übertragung auf die Hälfte reduziert ist wird als ED₅₀-Wert angegeben.

**Tabelle B**

| ED₅₀-Werte für ex vivo-Aktivität | | |
|---|---|---|
| **Beispiel-Nr.** | **ED**_{**50**} | **% Hemmung bei 10 mg/kg** |
| 12 | > 10 mg/kg | 49,5 % |
| 20 | < 10 mg/kg | 50,4 % |
| 21 | > 10 mg/kg | 34,4 % |

### In vivo Aktivität der erfindungsgemäßen Verbindungen

Bei Versuchen zur Bestimmung der oralen Wirkung auf Lipoproteine und Triglyceride wird syrischen Goldhamstern aus werkseigener Zucht Testsubstanz in DMSO gelöst und 0,5% Tylose suspendiert mittels Schlundsonde peroral verabreicht. Zur Bestimmung der CETP-Aktivität wird vor Versuchsbeginn durch retro-orbitale Punktion Blut entnommen (ca. 250 µl). Anschließend werden die Testsubstanzen peroral mittels einer Schlundsonde verabreicht. Die Kontrolltiere erhalten identische Volumen Lösemittel ohne Testsubstanz. Anschließend wird den Tieren das Futter entzogen und zu verschiedenen Zeitpunkten - bis zu 24 Stunden nach Substanzapplikation - durch Punktion des retroorbitalen Venenplexus Blut entnommen.
Durch Inkubation von 4°C über Nacht wird die Gerinnung abgeschlossen, anschließend wird 10 Minunten bei 6000 x g zentrifugiert. Im so erhaltenen Serum wird der Gehalt an Cholesterin und Triglyceriden mit Hilfe modifizierter kommerziell erhältlicher Enzymtests bestimmt (Cholesterin enzymatisch 14366 Merck, Triglyceride 14364 Merck). Serum wird in geeigneter Weise mit physiologischer Kochsalzlösung verdünnt.
100 µl Serum-Verdünnung werden mit 100 µl Testsubstanz in 96-Lochplatten versetzt und 10 Minuten bei Raumtemperatur inkubiert. Anschließend wird die optische Dichte bei einer Wellenlänge von 492 nm mit einem automatischen Platten-Lesegerät bestimmt. Die in den Proben enthaltene Triglycerid- bzw. Cholesterinkonzentration wird mit Hilfe einer parallel gemessenen Standardkurve bestimmt.
Die Bestimmung des Gehaltes von HDL-Cholesterin wird nach Präzipitation der ApoB-haltigen Lipoproteine mittels eines Reagenziengemisch (Sigma 352-4 HDL Cholesterol Reagenz) nach Herstellerangaben durchgeführt.

**Tabelle C**

| HDL-Anstieg bei in vivo-Versuchen | | |
|---|---|---|
| **Beispiel-Nr.** | Dosis [mg/kg] | % HDL-Anstieg |
| 12 | 2 x 3 | 12,37 |
| 20 | 2 x 3 | 9,21 |

### In vivo Wirksamkeit an transgenen hCETP-Mäusen

Transgenen Mäusen aus eigener Zucht (Dinchuck, Hart, Gonzalez, Karmann, Schmidt, Wirak; BBA (1995), 1295, 301) wurden die zu prüfenden Substanzen im Futter verabreicht. Vor Versuchsbeginn wurde den Mäusen retroorbital Blut entnommen, um Cholesterin und Triglyceride im Serum zu bestimmen. Das Serum wurde wie oben für Hamster beschrieben durch Inkubation bei 4°C über Nacht und anschließender Zentrifugation bei 6000 x g gewonnen. Nach einer Woche wurde den Mäusen wieder Blut entnommen, um Lipoproteine und Triglyceride zu bestimmen. Die Veränderung der gemessenen Parameter werden als prozentuale Veränderung gegenüber dem Ausgangswert ausgedrückt.

**Tabelle D**

| **Beispiel-Nr.** | **HDL** | **LDL** | **Triglyceride** |
|---|---|---|---|
| 20 (80 ppm) | + 14,5 % | + 6,7 % | -24,5 % |

Die Erfindung betrifft außerdem die Kombination von heterocyclisch annellierten Pyridinen der allgemeinen Formel (I) mit einem Glucosidase- und/oder Amylasehemmer zur Behandlung von familiärer Hyperlipidaemien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Weiterhin können die erfindungsgemäßen Verbindungen in Kombination mit Cholesterin senkenden Vastatinen oder Apo B-senkenden Prinzipien kombiniert werden, um Dyslipidemien, kombinierte Hyperlipidemien, Hypercholesterolemien oder Hypertriglyceridemien zu behandeln.

Die genannten Kombinationen sind auch zur primären oder sekundären Prävention koronarer Herzerkrankungen (z.B. Myokardinfarkt) einsetzbar.

Vastatine im Rahmen der Erfindung sind beispielsweise Lovastatin, Simvastatin, Pravastatin, Fluvastatin, Atorvastatin und Cerivastatin. Apo B-senkende Mittel sind zum Beispiel MTP-Inhibitoren. Bevorzugt ist die Kombination von Cerivastatin oder Apo B-Inhibitoren mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise intravenös, parenteral, perlingual oder vorzugsweise oral.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Verwendete Abkürzungen:

- DAST =: Dimethylaminoschwefeltrifluorid
- PTS =: para-Toluolsulfonsäure
- PDC =: Pyridiniumdichromat
- PE/EE =: Petrolether / Essigsäureethylester
- THF =: Tetrahydrofuran
- Tol/EE =: Toluol/Essigsäureethylester

### Beispiel I:

6-Cyclopentyl-4-(4-fluorphenyl)-2-oxo-1,2,3,4-tetrahydropyridin-3,5-dicarbonsäure-3-ethylester-5-methylester

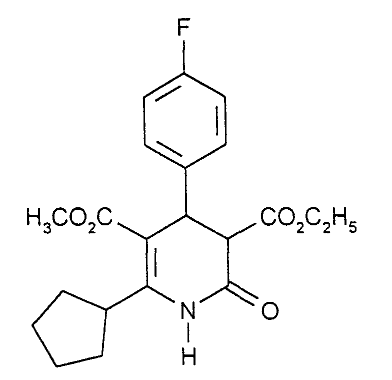

50 g (300 mmol) 3-Amino-3-cyclopentyl-prop-2-en-carbonsäuremethylester, 78,7 g (300 mmol) 2-(4-Fluorbenzyliden)-malonsäure-diethylester, 1,1 g (20 mmol) Natriummethylat und 6 ml Ethanol werden 80 Stunden unter Rühren auf 140°C erhitzt. Nach Abkühlen auf Raumtemperatur werden 500 ml Essigsäureethylester zugegeben und die Lösung nacheinander mit je 100 ml Wasser und gesättigter Kochsalzlösung gewaschen. Die organische Phase wird über Natriumsulfat getrocknet. Nach Einengen im Vakuum wird der Rückstand in 300 ml Cyclohexan unter Rühren 10 min zum Sieden erhitzt und danach langsam auf Raumtemperatur abgekühlt. Der ausgefallene Feststoff wird im Vakuum bis zur Gewichtskonstanz getrocknet.
Ausbeute: 74,9 g (65 % d. Th.)
R_{f} = 0,41 (PE/EE 4:1)

### Beispiel II:

6-Cyclopentyl-4-(4-fluorphenyl)-2-oxo-1,2-dihydropyridin-3,5-dicarbonsäure-3-ethylester-5-methylester

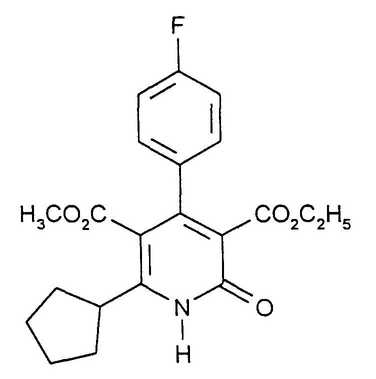

38 g (98 mmol) der Verbindung aus Beispiel I werden in 300 ml Acetonitril gelöst und innerhalb 1 Stunde bei Raumtemperatur mit 120 g Cer-(IV)-ammoniumnitrat, gelöst in 300 ml Wasser, versetzt. Anschließend wird 3 Stunden bei Raumtemperatur gerührt, wobei ein weißer Feststoff ausfällt. Zur Vervollständigung der Kristallisation läßt man 16 Stunden bei 5°C stehen. Der Feststoff wird abgesaugt und in kleinen Portionen mit 100 ml Wasser gewaschen. Der weiße Feststoff wird im Vakuum 2 Tage getrocknet.
Ausbeute: 31,6 g (83 % d. Th.)
R_{f} = 0,28 (PE/EE 4:1)

### Beispiel III:

2-Chlor-6-cyclopentyl-4-(4-fluorphenyl)-pyridin-3,5-dicarbonsäure-3-ethylester-5-methylester

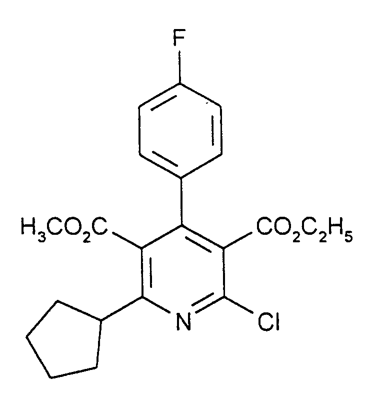

30,5 g (79 mmol) der Verbindung aus Beispiel II werden in 80 ml Phosphoroxychlorid 18 Stunden bei 110°C gerührt. Nach Abkühlen auf Raumtemperatur entfernt man das Phosphoroxychlorid im Vakuum. Der Rückstand wird unter Eiskühlung mit gesättigter Natriumhydrogencarbonatlösung neutralisiert und zweimal mit je 150 ml Dichlormethan extrahiert. Die vereinigten organischen Phasen werden über Natriumsulfat getrocknet und durch 250 g Kieselgel (230-400 mesh) filtriert. Es wird noch zweimal mit je 250 ml Dichlormethan nachgewaschen. Nach Einengen im Vakuum wird das Produkt im Hochvakuum getrocknet.
Ausbeute: 24,8 g (77 % d. Th.)
R_{f} = 0,78 (PE/EE 4:1)

### Beispiel IV:

2-Benzylamino-6-cyclopentyl-4-(4-fluorphenyl)-pyridin-3,5-dicarbonsäure-3-ethylester-5-methylester

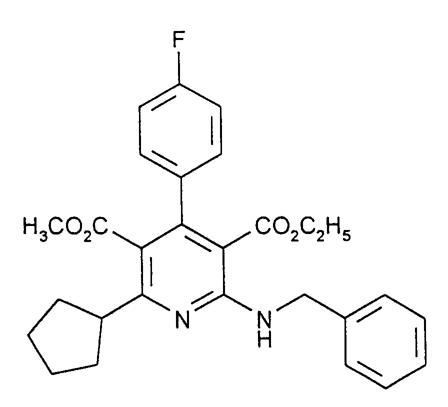

26 g (64 mmol) der Verbindung aus Beispiel III, 14 ml (130 mmol) Benzylamin und 17 g (160 mmol) Natriumcarbonat werden in 220 ml Acetonitril 2 Tage unter Rückfluß gerührt. Es werden noch 6,9 ml (64 mmol) Benzylamin sowie 6,8 g (64 mmol) Natriumcarbonat hinzugefügt und weitere 20 Stunden unter Rückfluß gerührt. Nach Abkühlen auf Raumtemperatur wird über Kieselgel abgesaugt und mit 100 ml Essigsäureethylester nachgewaschen. Nach Einengen im Vakuum nimmt man den teilweise kristallisierenden Rückstand in 100 ml Petrolether unter Rühren auf. Der ausgefallene Feststoff wird abgesaugt, mit etwas Petrolether gewaschen und im Hochvakuum getrocknet. Die verbleibende Mutterlauge wird eingeengt und über Kieselgel chromatographiert (200 g Kieselgel 230-400 mesh, d = 3,5 cm, Laufmittel Toluol).
Ausbeute: 25,1 g (82 % d. Th.)
R_{f} = 0,54 (PE/EE 8:1)

### Beispiel V:

1-Benzyl-7-cyclopentyl-5-(4-fluorphenyl)-4-oxo-1,2,3,4-tetrahydro-[1,8]-naphthyridin-3,6-dicarbonsäure-3-ethylester-6-methylester

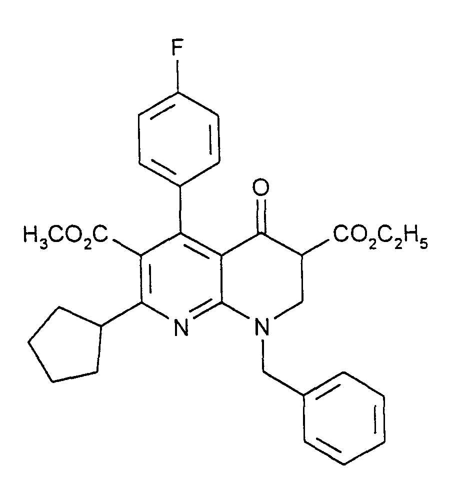

Unter Argon werden 8,1 g (17 mmol) der Verbindung aus Beispiel IV in 60 ml absolutem THF gelöst. Zu dieser Lösung werden bei 0°C 2,4 g (21,2 mmol) Kalium-tert.butylat gegeben und 15 min bei dieser Temperatur gerührt. Anschließend werden bei 0°C 2,30 ml (21,2 mmol) Acrylsäureethylester zugetropft und 16 Stunden bei Raumtemperatur gerührt. Zu dieser Lösung werden nochmals 0,39 g (3,4 mmol) Kalium-tert.butylat sowie 0,37 g (3,4 mmol) Acrylsäureethylester hinzugefügt. Es wird weitere 20 Stunden bei Raumtemperatur gerührt. Anschließend gibt man das Reaktionsgemisch auf 100 ml Eiswasser und säuert mit 20 ml 2 molarer Salzsäure an. Es wird zweimal mit je 100 ml Essigsäureethylester extrahiert, die vereinigten organischen Phasen über Natriumsulfat getrocknet und im Vakuum eingeengt. Der Rückstand wird über Kieselgel chromatographiert (250 g Kieselgel 230-400 mesh, d = 3,5 cm, Laufmittel Petrolether/Essigsäureethylester 12:1).
Ausbeute: 7,64 g (90 % d. Th.)
R_{f} = 0,38 (PE/EE 8:1)

### Beispiel VI:

8-Benzyl-2-cyclopentyl-4-(4-fluorphenyl)-5-oxo-5,6,7,8-tetrahydro-[1,8]-naphthyridin-3-carbonsäuremethylester

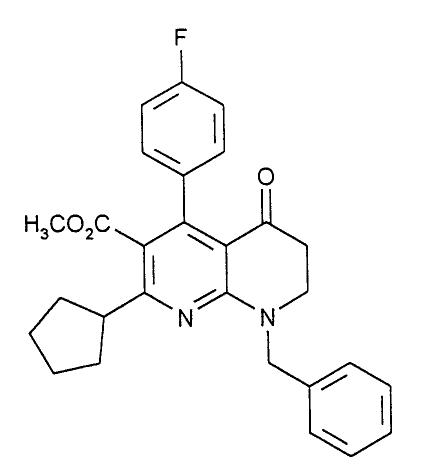

7,64 g (15,32 mmol) der Verbindung aus Beispiel V werden in Ethanol unter Zusatz von 9,8 ml (36,78 mmol) 15 %iger Natronlauge 3 Stunden unter Rückfluß erhitzt. Nach Abkühlen auf Raumtemperatur wird im Vakuum eingeengt und der Rückstand mit je 50 ml Dichlormethan und Wasser versetzt. Die wässrige Phase wird mit 50 ml Dichlormethan extrahiert und die vereinigten organischen Phasen über Natriumsulfat getrocknet. Nach Einengen im Vakuum wird der verbleibende Rückstand über Kieselgel chromatographiert (250 g Kieselgel 230-400 mesh, d = 3,5 cm, Laufmittel Petrolether/Essigsäureethylester 8:1).
Ausbeute: 5,74 g (82 % d. Th.)
R_{f} = 0,52 (PE/EE 4:1)

### Beispiel VII:

8-Benzyl-2-cyclopentyl-5,5-(1,2-ethandioxy)-4-(4-fluorphenyl)-5,6,7,8-tetrahydro-[1,8]-naphthyridin-3-carbonsäuremethylester

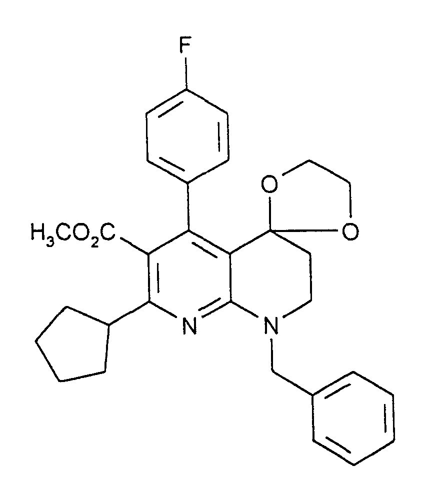

5,70 g (12,4 mmol) der Verbindung aus Beispiel VI, 10 ml 1,2-Ethandiol und 0,15 g (0,79 mmol) p-Toluolsulfonsäure werden in 200 ml Benzol 20 Stunden unter Rückfluß am Wasserabscheider erhitzt. Nach Abkühlen auf Raumtemperatur versetzt man mit 50 ml gesättigter Natriumhydrogencarbonatlösung. Die organische Phase wird einmal mit Wasser gewaschen, über Natriumsulfat getrocknet und im Vakuum eingeengt. Der verbliebene Feststoff wird aus Pentan/Diethylether umkristallisiert.
Ausbeute: 5,47 g (88 % d. Th.)
R_{f} = 0,37 (PE/EE 8:1)

### Beispiel VIII:

1-Benzyl-7-cyclopentyl-4,4-(1,2-ethandioxy)-5-(4-fluorphenyl)-6-hydroxymethyl-1,2,3,4-tetrahydro-[1,8]-naphthyridin

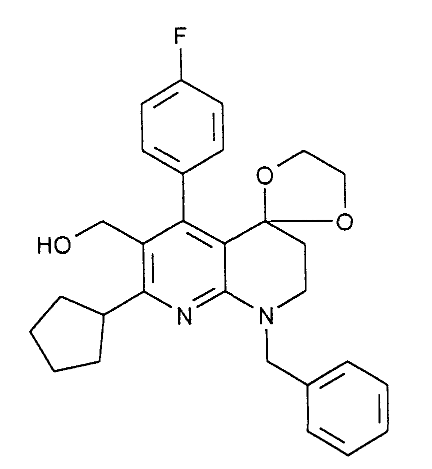

Unter Argon werden 5,47 g (10,90 mmol) der Verbindung aus Beispiel VII in 40 ml absolutem THF gelöst. Zu dieser Lösung werden bei 0°C 21,79 ml Lithiumaluminiumhydrid (1 molare Lösung in THF) getropft. Anschließend wird 20 Stunden bei Raumtemperatur gerührt und dann auf 0°C gekühlt. Bei dieser Temperatur werden 40 ml Wasser zugegeben. Die wässrige Phase wird zweimal mit je 40 ml Essigsäureethylester extrahiert. Die vereinigten organischen Phasen werden einmal mit 40 ml gesättigter Kochsalzlösung gewaschen, über Natriumsulfat getrocknet und im Vakuum eingeengt. Der Rückstand wird über Kieselgel chromatographiert (250 g Kieselgel 230-400 mesh, d = 3,5 cm, Laufmittel Petrolether/Essigsäureethylester 8:1).
Ausbeute: 3,55 g (69 % d. Th.)
R_{f} = 0,46 (PE/EE 4:1)

### Beispiel IX:

8-Benzyl-2-cyclopentyl-5,5-(1,2-ethandioxy)-4-(4-fluorphenyl)-5,6,7,8-tetrahydro-[1,8]-naphthyridin-3-carbaldehyd

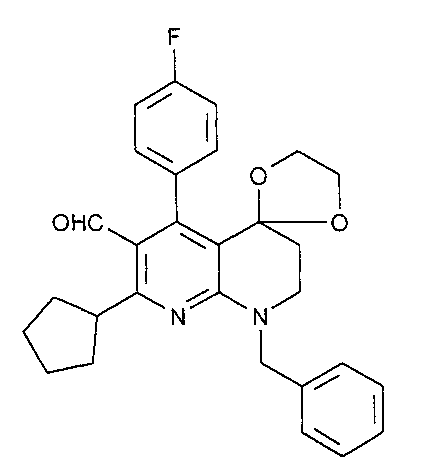

Unter Argon werden 3,20 g (6,74 mmol) der Verbindung aus Beispiel VIII in 30 ml absolutem Dichlormethan gelöst. Zu dieser Lösung werden bei -10°C 3,24 g (8,43 mmol) Pyridiniumdichromat in kleinen Portionen eingetragen. Nach 5 Stunden Rühren bei Raumtemperatur wird das Reaktionsgemisch über 20 g Kieselgel (230-400 mesh) abgesaugt, zweimal mit je 30 ml Dichlormethan gewaschen und im Vakuum eingeengt.
Ausbeute: 1,39 g (44 % d. Th.)
R_{f} = 0,66 (PE/EE 4:1)

### Beispiel X:

1-Benzyl-7-cyclopentyl-5-(4-fluorphenyl)-4-oxo-1,4-dihydro-[1,8]-naphthyridin-3,6-dicarbonsäure-3-ethylester-6-methylester

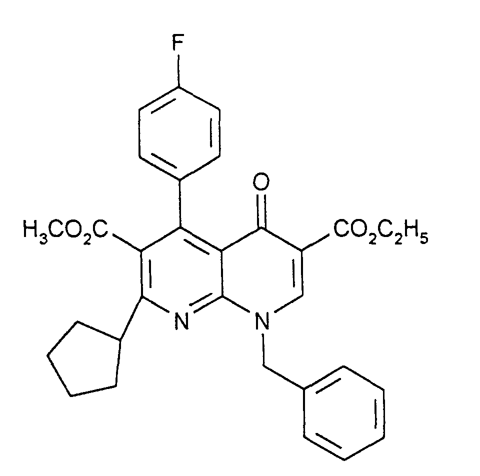

12,5 g (23,7 mmol) der Verbindung aus Beispiel V und 20 g (230 mmol) Mangandioxid werden in 250 ml Essigsäureethylester 2,5 Stunden unter Rückfluß kräftig gerührt. Nach Abkühlen auf Raumtemperatur wird durch Kieselgur filtriert und im Vakuum eingeengt.
Ausbeute: 10,75 g (86 % d. Th.)
R_{f} = 0,40 (PE/EE 2:1)

### Beispiel XI:

1-Benzyl-7-cyclopentyl-5-(4-fluorphenyl)-2-methyl-4-oxo-1,2,3,4-tetrahydro-[1,8]-naphthyridin-3,6-dicarbonsäure-3 -ethylester-6-methylester

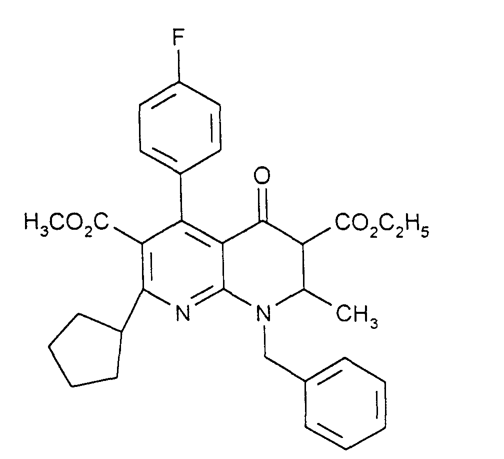

5,0 g (9,46 mmol) der Verbindung aus Beispiel X werden unter Argon in 60 ml absolutem THF gelöst. Bei -78°C werden 3,5 ml Methylmagnesiumbromidlösung (3 molare Lösung in Diethylether) innerhalb von 30 min zugetropft. Es wird 2 Stunden bei -78°C und anschließend 1 Stunde bei -50°C gerührt. Das Reaktionsgemisch wird mit 30 ml Phosphatpufferlösung (pH 7) versetzt und auf Raumtemperatur erwärmt. Nach Abtrennen der organischen Phase extrahiert man die wässrige Phase einmal mit 50 ml Essigsäureethylester. Die vereinigten organischen Phasen werden über Natriumsulfat getrocknet und im Vakuum eingeengt. Der Rückstand wird über Kieselgel chromatographiert (200 g Kieselgel 230-400 mesh, d = 3,5 cm, Laufmittel Petrolether/Essigsäureethylester 10:1).
Ausbeute: 2,25 g (Diastereomerengemisch) (46 % d. Th.)
R_{f} = Dia A: 0,80 (PE/EE 8:1)
R_{f} = Dia B: 0,69 (PE/EE 8:1)

### Beispiel XII:

[Benzyl-(2-oxo-propyl)-amino]-essigsäure-ethylester

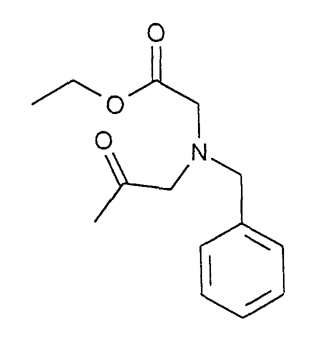

96,6 g (500 mmol) Benzylamino-essigsäureethylester und 42,0 g (500 mmol) Natriumhydrogencarbonat werden in 400 ml Ethanol auf 55°C erhitzt und innerhalb 1 Stunde mit 46,6 g (500 mmol) Chloraceton versetzt. Es wird 18 Stunden bei 60°C gerührt und anschließend auf Raumtemperatur abgekühlt. Der Feststoff wird abgesaugt und mit 100 ml Ethanol gewaschen. Das Filtrat wird im Vakuum eingeengt und der verbliebene Rückstand unter Eiskühlung mit 125 ml 1 molarer Natronlauge versetzt. Anschließend werden 17,4 g (124 mmol) Benzoylchlorid zugegeben und 10 min kräftig gerührt. Mit 1 molarer Salzsäure wird auf pH 1,5 angesäuert und dreimal mit je 50 ml Diethylether extrahiert. Die vereinigten organischen Phasen werden zweimal mit jeweils 50 ml 1 molarer Salzsäure gewaschen. Die vereinigten wässrigen Phasen werden mit 1 molarer Natronlauge auf pH 7,5 gestellt und dreimal mit je 100 ml Diethylether extrahiert. Die vereinigten organischen Phasen werden über Natriumsulfat getrocknet und im Vakuum eingeengt.
Ausbeute: 77,3 g (62 % d. Th.)
R_{f} = 0,36 (PE/EE 4:1)

### Beispiel XIII:

1-Benzyl-piperidin-3,5-dion

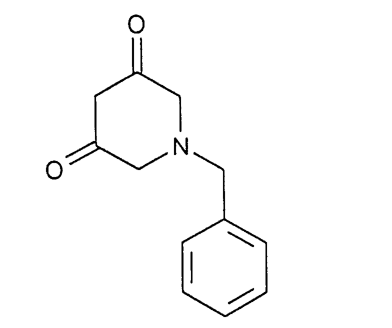

Unter Argon werden 38,3 g (341 mmol) Kalium-tert.butylat in 500 ml absolutem Diethylether und 500 ml tert.Butanol gelöst. Anschließend gibt man bei -15°C eine Lösung von 77,3 g (310 mmol) der Verbindung aus Beispiel XII in 100 ml Diethylether hinzu und rührt 18 Stunden bei Raumtemperatur. Bei einer Badtemperatur von 30°C engt man im Vakuum nicht ganz bis zur Trockene ein und nimmt den Rückstand unter Argon mit 500 ml absolutem Diethylether auf. Der Feststoff wird abgesaugt und mit 100 ml absolutem Diethylether gewaschen. Anschließend löst man den Feststoff in 260 ml 2 molarer Essigsäure. Nach Zugabe von 90 ml Wasser fällt das gewünschte Produkt aus. Zur Vervollständigung der Kristallisation wird das Gemisch 18 Stunden bei 5°C stehen gelassen. Der Feststoff wird abgesaugt, mit 50 ml Eiswasser gewaschen und 24 Stunden über Phosphorpentoxid im Vakuum getrocknet.
Ausbeute: 46,1 g (73 % d. Th.)
R_{f} = 0,36 (Dichlormethan/Methanol/Eisessig 3:1:0,1)

### Beispiel XIV:

7-Benzyl-2-cyclopentyl-4-(4-fluorphenyl)-5-oxo-1,4,5,6,7,8-hexahydro-[1,7]-naphthyridin-3-carbonsäuremethylester

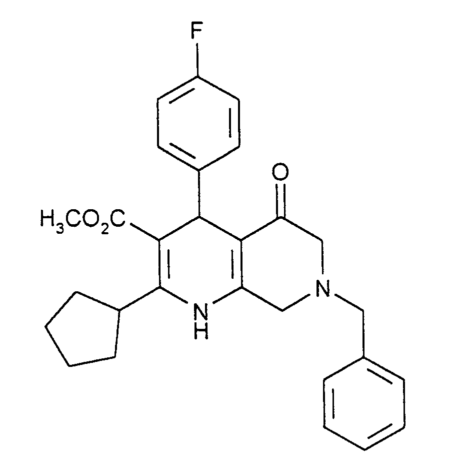

15,4 g (76 mmol) der Verbindung aus Beispiel XIII, 9,4 g (76 mmol) 4-Fluorbenzaldehyd und 12,8 g (76 mmol) 3-Amino-3-cyclopentyl-prop-2-encarbonsäuremethylester werden in 150 ml Ethanol 18 Stunden unter Rückfluß erhitzt. Anschließend wird im Vakuum eingeengt und der Rückstand über Kieselgel chromatographiert (500 g Kieselgel 230-400 mesh, d = 4,5 cm, Laufmittel Toluol/Essigsäureethylester 7:3).
Ausbeute: 11,3 g (32 % d. Th.)
R_{f} = 0,11 (PE/EE 4:1)

### Beispiel XV:

7-Benzyl-2-cyclopentyl-4-(4-fluorphenyl)-5-oxo-5,6,7,8-tetrahydro-[1,7]-naphthyridin-3-carbonsäuremethylester

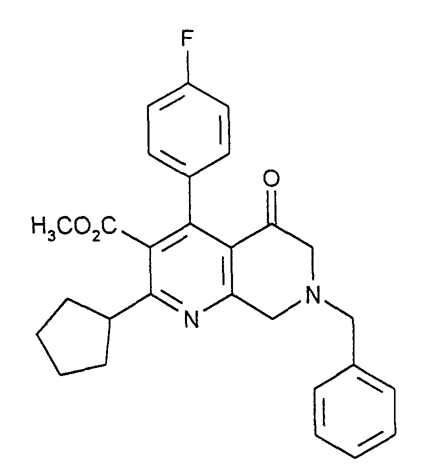

11,2 g (24 mmol) der Verbindung aus Beispiel XIV werden in 300 ml absolutem Dichlormethan gelöst und mit 6,1 g (26,7 mmol) 2,3-Dichlor-5,6-dicyan-pbenzochinon versetzt. Es wird 3 Stunden bei Raumtemperatur gerührt und anschließend über 400 g Kieselgel (230-400 mesh) abgesaugt. Das Kieselgel wird mit 5 1 Dichlormethan nachgewaschen und die vereinigten organischen Phasen im Vakuum eingeengt.
Ausbeute: 7,9 g (71 % d. Th.)
R_{f} = 0,50 (PE/EE 6:1)

### Beispiel XVI:

7-Benzyl-2-cyclopentyl-4-(4-fluorphenyl)-5-hydroxy-5,6,7,8-tetrahydro-[1,7]-naphthyridin-3-carbonsäuremethylester

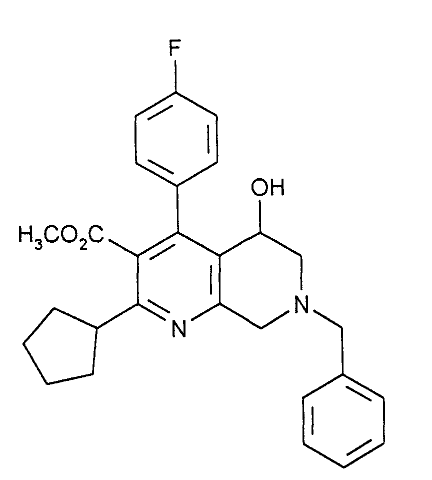

Unter Argon werden 5,5 g (12 mmol) der Verbindung aus Beispiel XV in 100 ml Methanol gelöst und bei 0°C mit 0,91 g (24 mmol) Natriumborhydrid versetzt. Es wird 20 min bei 0°C und anschließend 1 Stunde bei Raumtemperatur gerührt. Die Reaktionslösung wird mit 100 ml gesättigter Ammoniumchloridlösung versetzt und dreimal mit je 100 ml Essigsäureethylester extrahiert. Die vereinigten organischen Phasen werden mit 50 ml gesättigter Kochsalzlösung gewaschen, über Natriumsulfat getrocknet und im Vakuum eingeengt. Der Rückstand wird über Kieselgel chromatographiert (400 g Kieselgel 230-400 mesh, d = 4,5 cm Laufmittel Petrolether/Essigsäureethylester 4:1).
Ausbeute: 4,6 g (83 % d. Th.)
R_{f} = 0,36 (PE/EE 6:1)

### Beispiel XVII:

7-Benzyl-5-tert.butyldimethylsiloxy-2-cyclopentyl-4-(4-fluorphenyl)-5,6,7,8-tetrahydro-[1,7]-naphthyridin-3-carbonsäuremethylester

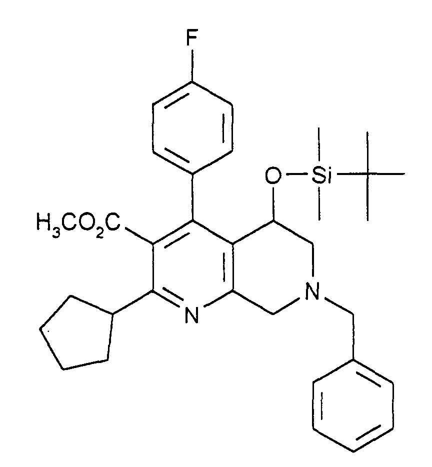

Unter Argon werden 4,6 g (10,0 mmol) der Verbindung aus Beispiel XVI in 40 ml absolutem DMF gelöst und bei Raumtemperatur mit 2,5 g (36 mmol) Imidazol, 3,0 g (20 mmol) tert.Butyldimethylchlorsilan und 0,02 g (0,2 mmol) Dimethylaminopyridin versetzt. Es wird 3 Tage bei Raumtemperatur gerührt und anschließend unter Rühren zwischen 100 ml gesättigter Ammoniumchloridlösung und 100 ml Toluol verteilt. Die wässrige Phase wird noch zweimal mit je 100 ml Toluol extrahiert, die vereinigten organischen Phasen zweimal mit 25 ml Wasser gewaschen, über Natriumsulfat getrocknet und im Vakuum eingeengt. Der Rückstand wird über Kieselgel chromatographiert (400 g Kieselgel 230-400 mesh, d = 4,5 cm, Laufmittel Petrolether/Essigsäureethylester 20:1).
Ausbeute: 5,7 g (99 % d. Th.)
R_{f} = 0,21 (PE/EE 20:1)

### Beispiel XVIII:

7-Benzyl-5-tert.butyldimethylsiloxy-2-cyclopentyl-4-(4-fluorphenyl)-3-hydroxymethyl-5,6,7,8-tetrahydro-[1,7]-naphthyridin

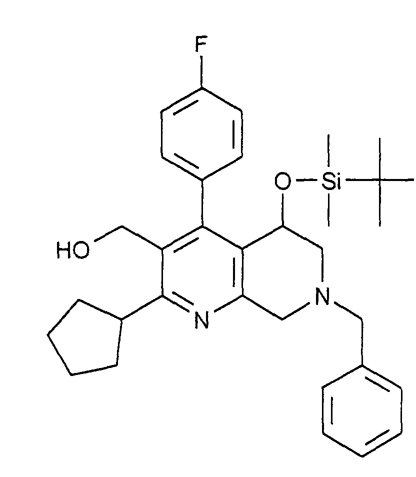

Unter Argon werden 3,0 g (5,2 mmol) der Verbindung aus Beispiel XVII in 60 ml absolutem Toluol gelöst. Bei -78°C werden 15,7 ml Diisobutylaluminiumhydridlösung (1 molar in Toluol) zugetropft und 18 Stunden bei dieser Temperatur gerührt. Anschließend werden bei -78°C 5 ml Methanol und danach 20 ml einer 20 %igen Natriumkaliumtartratlösung zugegeben. Es wird auf Raumtemperatur erwärmt und zweimal mit je 50 ml Essigsäureethylester extrahiert. Die vereinigten organischen Phasen werden je einmal mit 20 ml Wasser und 20 ml gesättigter Kochsalzlösung gewaschen, über Natriumsulfat getrocknet und im Vakuum eingeengt. Der Rückstand wird über Kieselgel chromatographiert (200 g Kieselgel 230-400 mesh, d = 3,5 cm, Laufmittel Petrolether/Essigsäureethylester 6:1).
Ausbeute: 1,67 g (59 % d. Th.)
R_{f} = 0,53 (PE/EE 4:1)

### Beispiel XIX:

7-Benzyl-5-tert.butyldimethylsiloxy-2-cyclopentyl-4-(4-fluorphenyl)-5,6,7,8-tetrahydro-[1,7]-naphthyridin-3-carbaldehyd

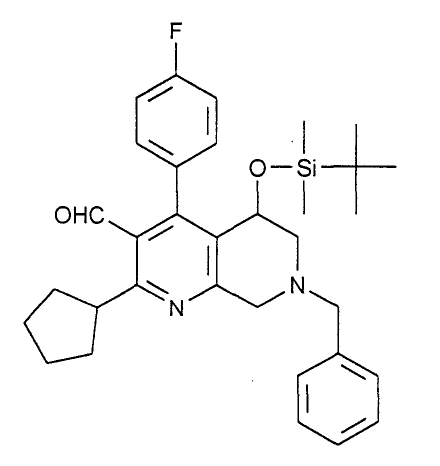

1,6 g (3,0 mmol) der Verbindung aus Beispiel XVIII werden in 15 ml absolutem Dichlormethan bei Raumtemperatur mit 1,5 g (15 mmol) Triethylamin und 4 ml Dimethylsulfoxid versetzt. Die Lösung wird auf 0°C gekühlt und mit 1,9 g (12 mmol) Schwefeltrioxid-Pyridin-Komplex versetzt. Es wird 3 Stunden bei 0°C gerührt und dann nochmals mit 1,5 g (15 mmol) Triethylamin, 4 ml Dimethylsulfoxid und 1,9 g (12 mmol) Schwefeltrioxid-Pyridin-Komplex versetzt. Die Lösung wird 1 Stunde bei 10°C gerührt und dann mit 20 ml Eiswasser versetzt. Nach Trennung der Phasen wird die wässrige Phase einmal mit 20 ml Dichlormethan extrahiert. Die vereinigten organischen Phasen werden mit jeweils 10 ml Wasser und gesättigter Kochsalzlösung gewaschen, über Natriumsulfat getrocknet und im Vakuum eingeengt. Der Rückstand wird über Kieselgel chromatographiert (200 g Kieselgel 230-400 mesh, d = 3,5 cm, Laufmittel Petrolether/Essigsäureethylester 8:1).
Ausbeute: 1,4 g (86 % d. Th.)
R_{f} = 0,91 (PE/EE 4:1)

### Herstellungsbeispiele:

### Beispiel 1:

1-Benzyl-7-cyclopentyl-4,4-(1,2-ethandioxy)-5-(4-fluorphenyl)-6-[hydroxy-(4-trifluormethyl-phenyl)-methyl]-1,2,3,4-tetrahydro-[1,8]-naphthyridin

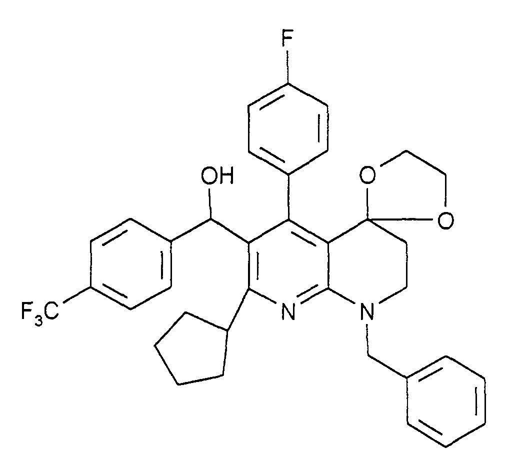

Unter Argon werden 0,49 g (20 mmol) Magnesiumspäne in 50 ml absolutem THF auf 60°C erhitzt und mit einer Lösung von 1,89 ml (13 mmol) 4-Brombenzotrifluorid in 8 ml absolutem THF innerhalb von 20 min versetzt. Es wird 2 Stunden unter Rückfluß erhitzt und anschließend auf Raumtemperatur abgekühlt (Grignard-Reagenz-Lösung). Eine Lösung von 1,39 g (2,94 mmol) der Verbindung aus Beispiel IX wird in 20 ml absolutem THF bei 0°C unter Argon mit 50 ml der Grignard-Reagenz-Lösung versetzt. Anschließend wird 1 Stunde bei Raumtemperatur gerührt. Das Reaktionsgemisch wird in 100 ml Phosphatpufferlösung (pH 7) und 100 ml Essigsäureethylester unter Rühren verteilt, die organische Phase abgetrennt und die wässrige Phase zweimal mit je 50 ml Essigsäureethylester extrahiert. Die vereinigten organischen Phasen werden über Natriumsulfat getrocknet und im Vakuum eingeengt. Der Rückstand wird über Kieselgel chromatographiert (200 g Kieselgel 230-400 mesh, d = 3,5 cm, Laufmittel Petrolether/Essigsäureethylester 6:1).
Ausbeute: 1,71 g (94 % d. Th.)
R_{f} = 0,42 (PE/EE 4:1)

### Beispiel 2:

1-Benzyl-7-cyclopentyl-5-(4-fluorphenyl)-6-[hydroxy-(4-trifluormethyl-phenyl)-methyl]-4-oxo-1,2,3,4-tetrahydro-[1,8]-naphthyridin

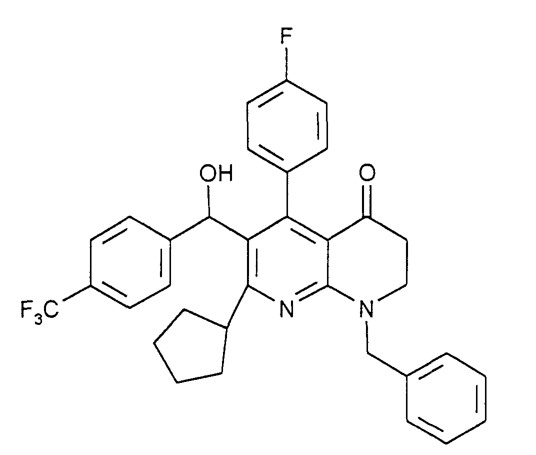

Eine Lösung von 1,71 g (2,76 mmol) der Verbindung aus Beispiel 1 in 40 ml absolutem Aceton wird bei Raumtemperatur mit 27 mg (0,14 mmol) p-Toluolsulfonsäure und 1 ml Wasser versetzt und 1 Stunde gerührt. Die Reaktionslösung wird über Natriumsulfat getrocknet und durch 30 g Kieselgel (230-400 mesh) filtriert Es wird mit 50 ml Dichlormethan nachgewaschen und die vereinigten organischen Filtrate im Vakuum eingeengt.
Ausbeute: 1,51 g (95 % d. Th.)
R_{f} = 0,32 (PE/BE 4:1)

### Beispiel 3:

1-Benzyl-7-cyclopentyl-5-(4-fluorphenyl)-6-[hydroxy-(4-trifluormethyl-phenyl)-methyl]-1,2,3,4-tetrahydro-[1,8]-naphthyridin-4-ol

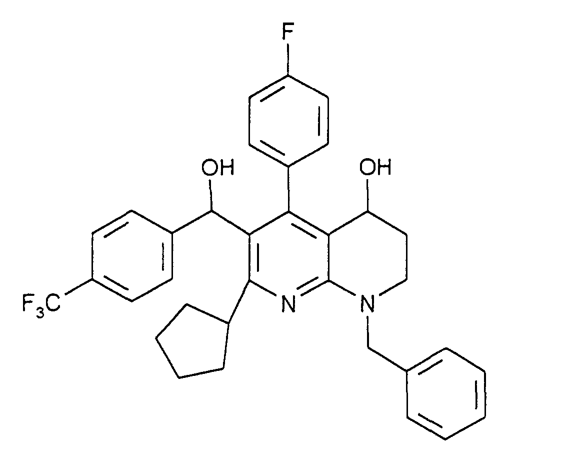

Unter Argon werden 0,80 g (1,39 mmol) der Verbindung aus Beispiel 2 in 30 ml absolutem THF gelöst und bei -78°C mit 4,17 ml Diisobutylaluminiumhydrid (1 molare Lösung in THF) versetzt. Es wird 1 Stunde bei -78°C gerührt und anschließend mit 10 ml gesättigter Natriumsulfatlösung versetzt. Nach Erwärmen auf Raumtemperatur wird die organische Phase abgetrennt und die wässrige Phase zweimal mit jeweils 30 ml Essigsäureethylester extrahiert. Die vereinigten organischen Phasen werden über Natriumsulfat getrocknet und im Vakuum eingeengt. Der Rückstand wird über Kieselgel chromatographiert (100 g Kieselgel 230-400 mesh, d = 2,5 cm, Laufmittel Cyclohexan/Essigsäureethylester 6:1).
Ausbeute Diastereomer A: 0,37 g (45 % d. Th.)
R_{f} = 0,46 (PE/EE 4:1)
Ausbeute Diastereomer B: 0,39 g (48 % d. Th.)
R_{f} = 0,19 (PE/EE 4:1)

### Beispiel 4:

7-Benzyl-5-tert.butyldimethylsiloxy-2-cyclopentyl-4-(4-fluorphenyl)-3-[hydroxy-(4-trifluormethyl-phenyl)-methyl]-5,6,7,8-tetrahydro-[1,7]-naphthydrin

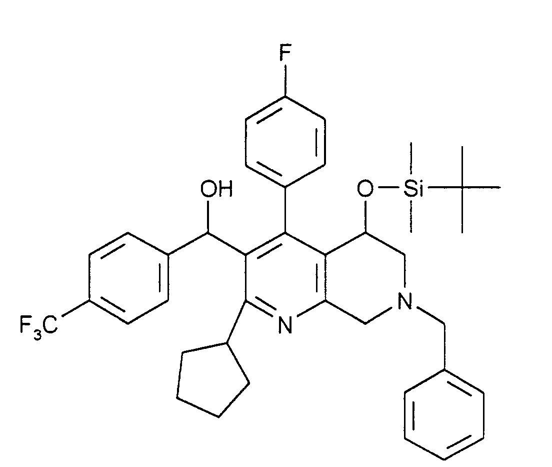

Unter Argon werden 0,21 g (7,7 mmol) Magnesiumspäne in 20 ml absolutem THF auf 60°C erhitzt und mit einer Lösung von 1,7 g (7,7 mmol) 4-Brombenzotrifluorid in 8 ml absolutem THF innerhalb von 20 min versetzt. Es wird 2 Stunden unter Rückfluß erhitzt und anschließend auf Raumtemperatur abgekühlt (Grignard-Reagenz-Lösung). Eine Lösung von 1,40 g (2,6 mmol) der Verbindung aus Beispiel XIX wird in 22 ml absolutem THF bei 0°C unter Argon mit 20 ml der Grignard-Reagenz-Lösung versetzt. Anschließend wird 1 Stunde bei Raumtemperatur gerührt. Das Reaktionsgemisch wird in 50 ml Phosphatpufferlösung (pH 7) und 50 ml Essigsäureethylester unter Rühren verteilt, die organische Phase abgetrennt und die wässrige Phase zweimal mit je 50 ml Essigsäureethylester extrahiert. Die vereinigten organischen Phasen werden über Natriumsulfat getrocknet und im Vakuum eingeengt. Der Rückstand wird über Kieselgel chromatographiert (250 g Kieselgel 230-400 mesh, d = 3,5 cm, Laufmittel Petrolether/Essigsäureethylester 6:1).
Ausbeute Dia A: 0,68 g (38 % d. Th.)
R_{f} (Dia A) = 0,55 (PE/EE 6:1)
Ausbeute Dia B: 0,50 g (28 % d. Th.)
R_{f} (Dia B) = 0,33 (PE/EE 6:1)

### Beispiel 5:

7-Benzyl-5-tert.butyldimethylsiloxy-2-cyclopentyl-4-(4-fluorphenyl)-3-[fluor-(4-trifluormethyl-phenyl)-methyl]-5,6,7,8-tetrahydro-[1,7]-naphthyridin

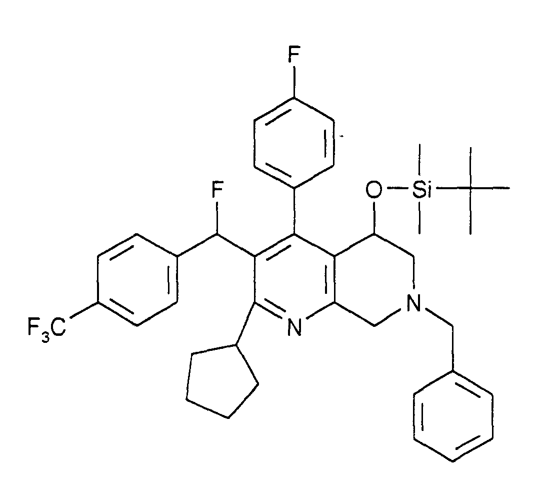

Unter Argon werden 200 mg (0,3 mmol) der Verbindung aus Beispiel 4 (Diastereomer A) in 6 ml absolutem Dichlormethan gelöst. Bei -30°C werden 0,06 ml (0,45 mmol) Diethylaminoschwefeltrifluorid zugetropft und noch 1 Stunde bei dieser Temperatur gerührt. Anschließend gibt man die Reaktionslösung auf 20 ml eiskalte, gesättigte Natriumhydrogencarbonatlösung. Es wird dreimal mit je 20 ml Dichlormethan extrahiert. Die vereinigten organischen Phasen werden über Natriumsulfat getrocknet und im Vakuum eingeengt.
Ausbeute: 193 mg (93 % d. Th.)
R_{f} = 0,82 (PE/EE 4:1)

### Beispiel 6:

7-Benzyl-2-cyclopentyl-4-(4-fluorphenyl)-3-[fluor-(4-trifluormethyl-phenyl)-methyl]-5,6,7,8-tetrahydro-[1,7]-naphthyridin-5-ol

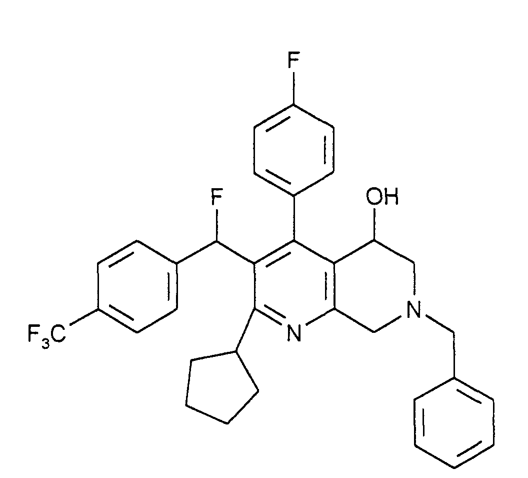

90 mg ( 0,13 mmol) der Verbindung aus Beispiel 5 werden in 3 ml THF und 3 ml Methanol bei Raumtemperatur gelöst. Zu dieser Lösung werden 1,3 ml einer 3 molaren Salzsäure gegeben und 18 Stunden bei Raumtemperatur gerührt. Das Reaktionsgemisch wird auf 10 ml gesättigte Natriumhydrogencarbonatlösung gegeben und dreimal mit je 20 ml Essigsäureethylester extrahiert. Die vereinigten organischen Phasen werden einmal mit 10 ml einer gesättigten Kochsalzlösung gewaschen, über Natriumsulfat getrocknet und im Vakuum eingeengt. Der Rückstand wird über Kieselgel chromatographiert (10 g Kieselgel 230-400 mesh, d = 2 cm), Laufmittel Petrolether/Essigsäureethylester 6:1).
Ausbeute: 28 mg (37 % d. Th.)
R_{f} = 0,60 (PE/EE 4:1)

In Analogie zu den oben aufgeführten Vorschriften werden die in den Tabellen 1, 2, 3, 4, 5 und 6 aufgeführten Verbindungen hergestellt.

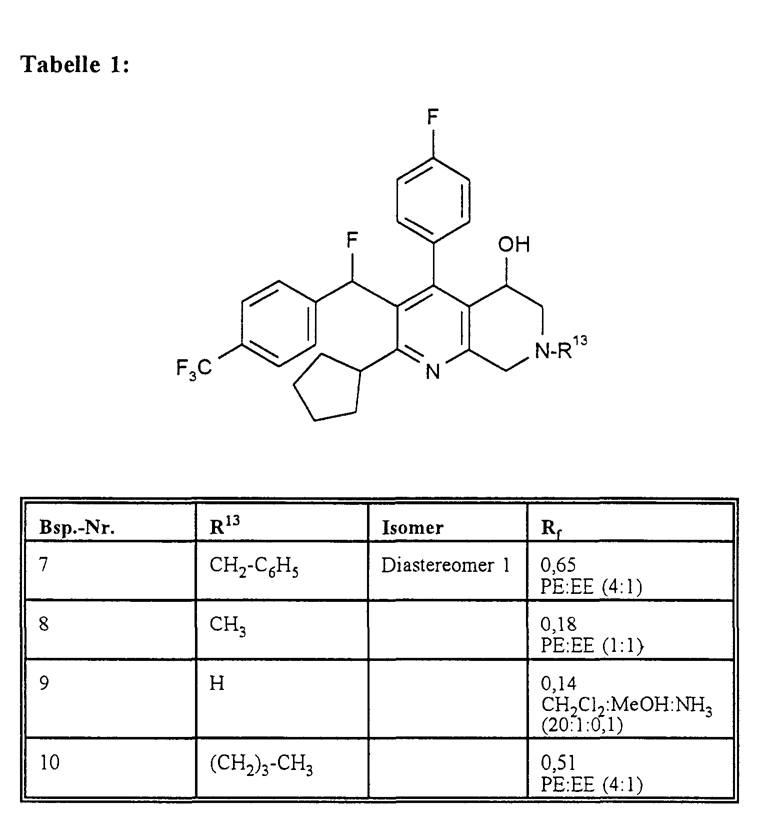

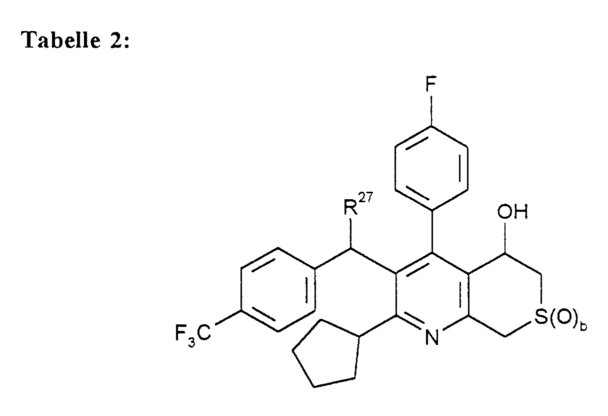

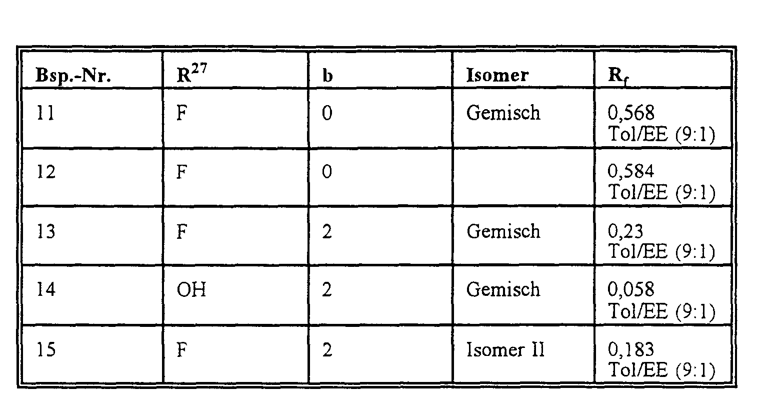

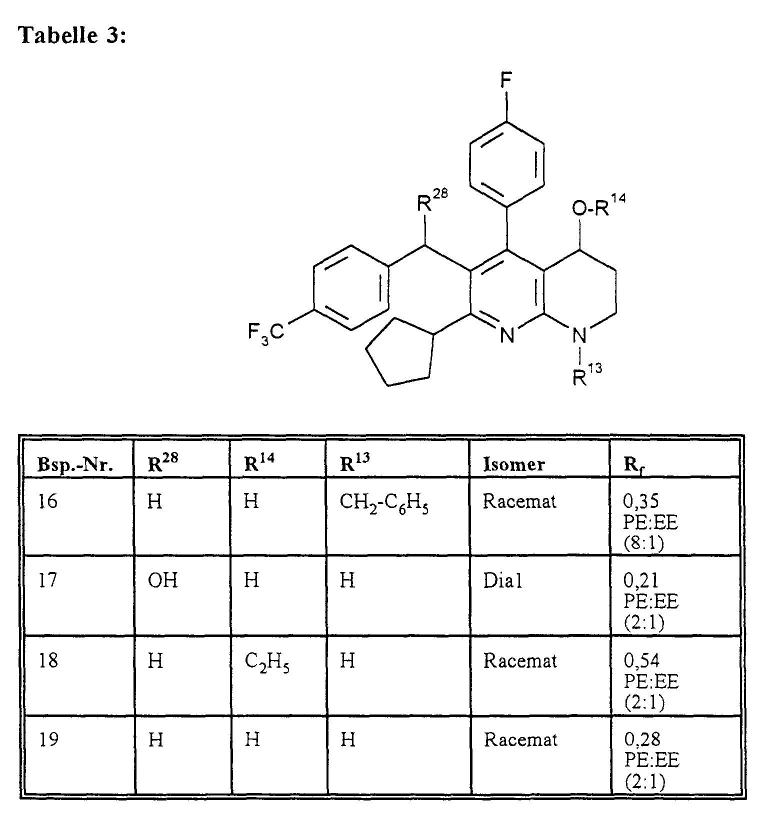

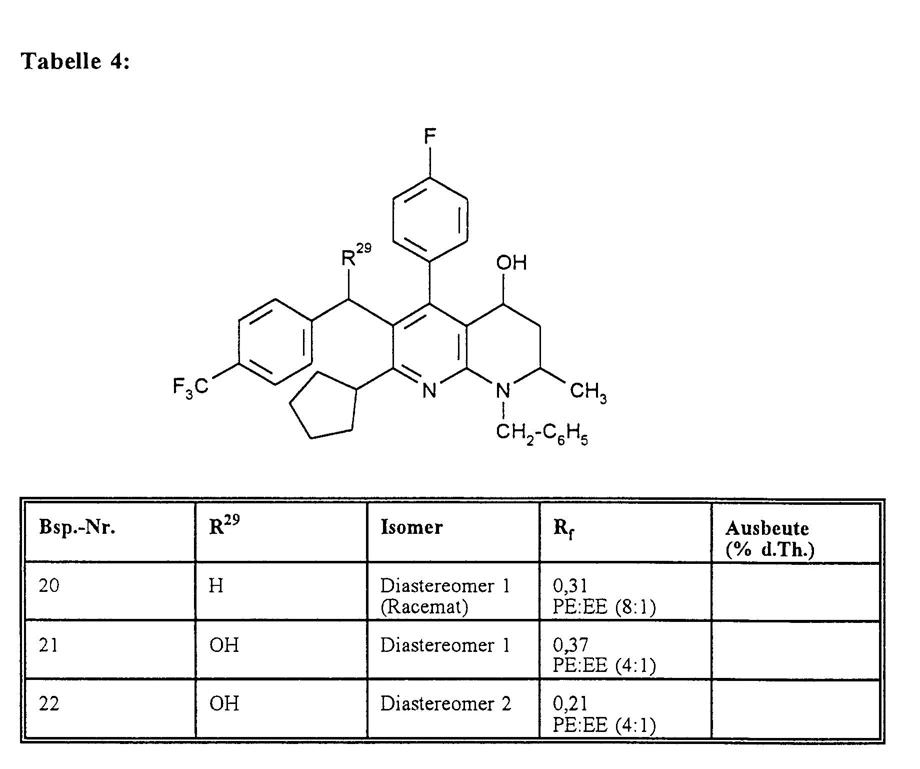

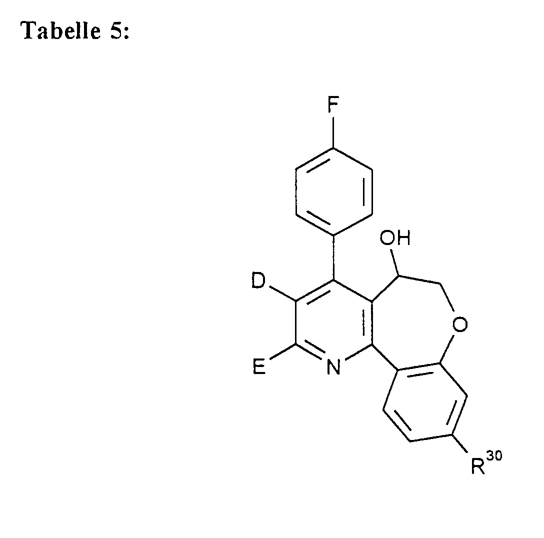

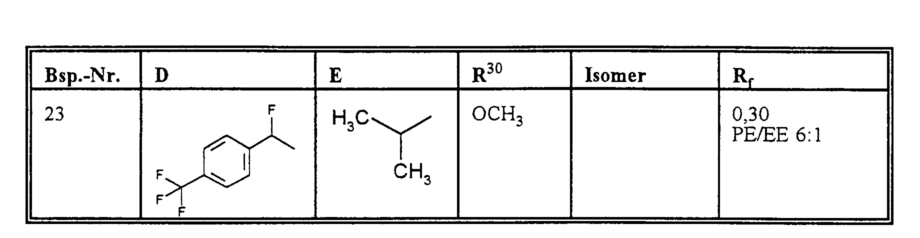

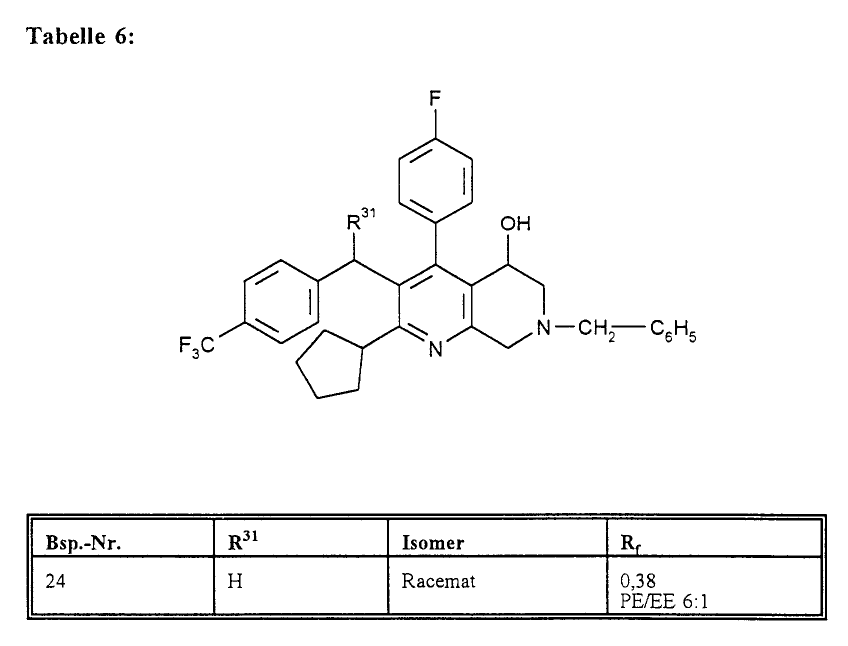

In Analogie zu den oben aufgeführten Vorschriften werden die in der Tabelle 7 aufgeführten Verbindungen hergestellt:

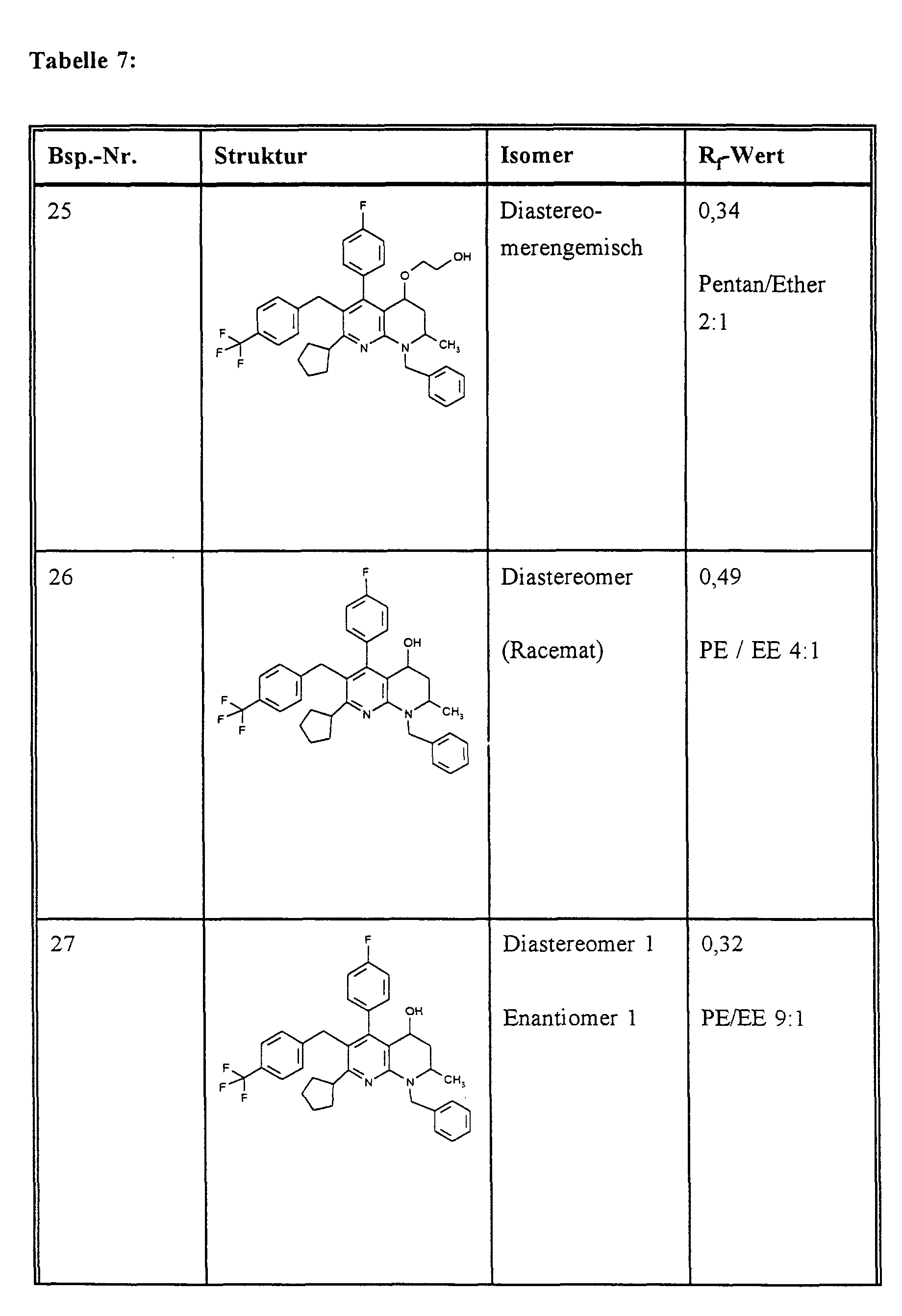

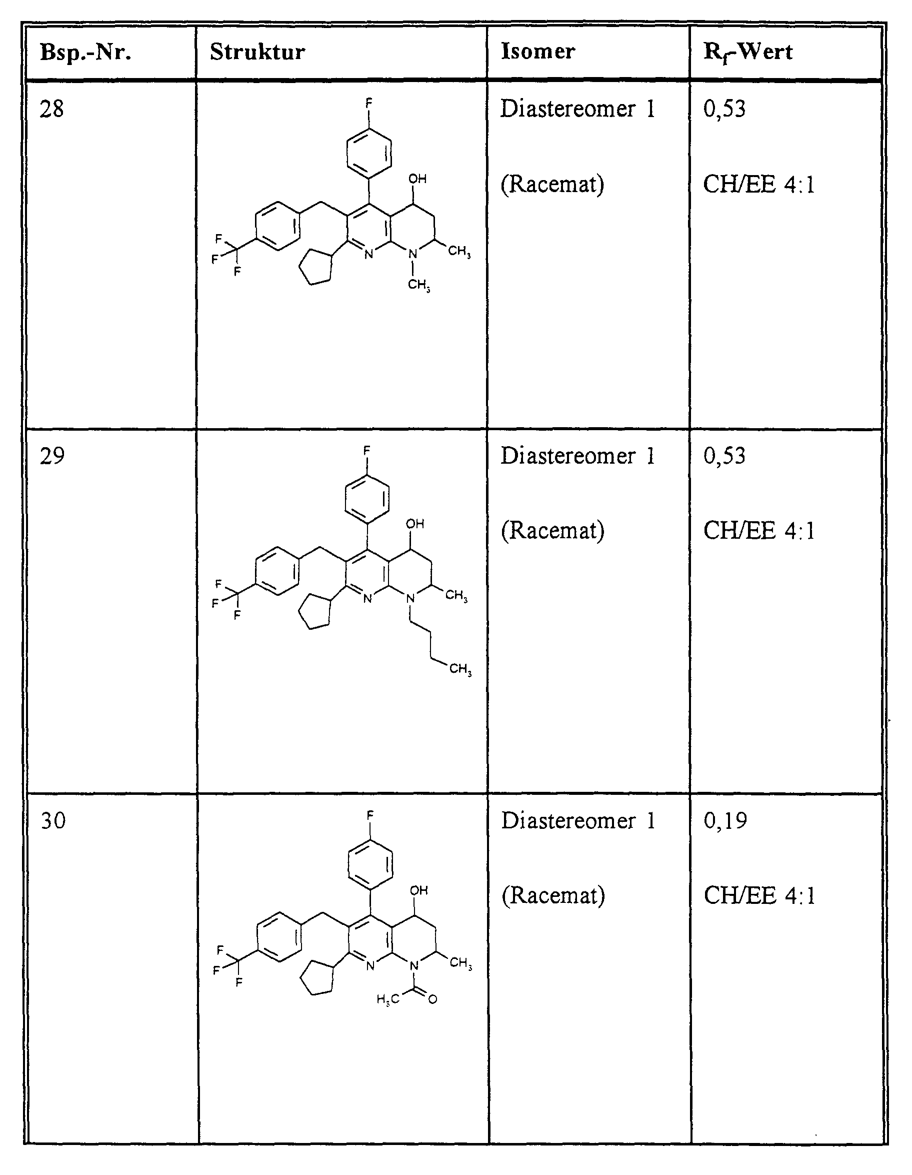

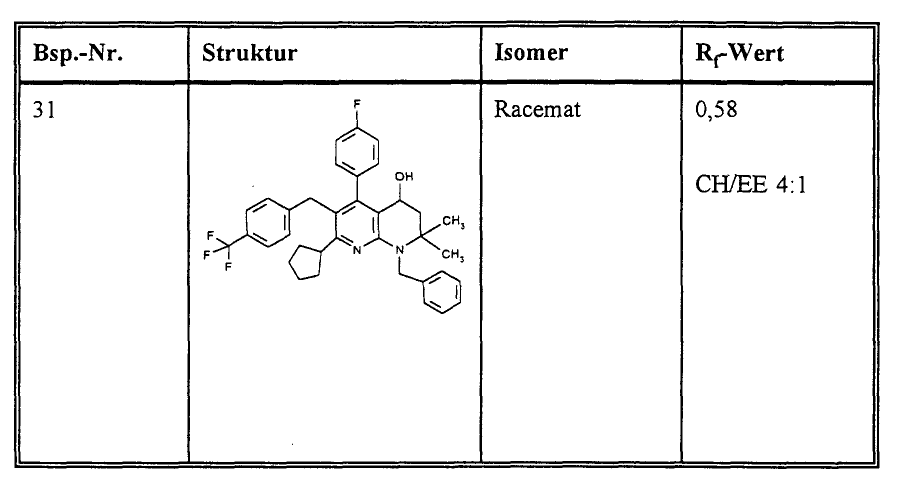

## Patentansprüche

1. Heterocyclisch kondensierte Pyridine der allgemeinen Formel (I), 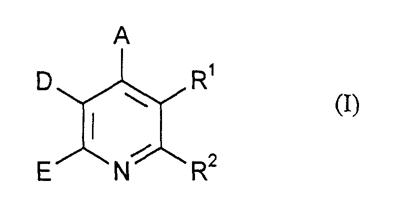 in welcher
A für Aryl mit 6 bis 10 Kohlenstoffatomen steht, das gegebenenfalls bis zu 5-fach gleich oder verschieden durch Halogen, Hydroxy, Trifluormethyl, Trifluormethoxy, Nitro oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR³R⁴ substituiert ist,
worin
R³ und R⁴ gleich oder verschieden sind und Wasserstoff, Phenyl oder geradkettiges oder verzweigtes Alkyl mit bis zu 6 Kohlenstoffatomen bedeuten,
D für einen Rest der Formel
R⁵―X―
oder 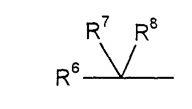 steht,
worin
R⁵ und R⁶ unabhängig voneinander Cycloalkyl mit 3 bis 8 Kohlenstoffatomen bedeuten, oder
Aryl mit 6 bis 10 Kohlenstoffatomen,
oder einen 5- bis 7-gliedrigen aromatischen, gegebenenfalls benzokondensierten Heterocyclus mit bis zu 3 Heteroatomen aus der Reihe S, N und/oder O bedeuten, die gegebenenfalls bis zu 5-fach gleich oder verschieden durch Trifluormethyl, Nitro, Trifluormethoxy, Halogen, Hydroxy, Carboxyl, durch geradkettiges oder verzweigtes Alkyl, Acyl, Alkoxy oder Alkoxycarbonyl mit jeweils bis zu 6 Kohlenstoffatomen oder durch Phenyl, Phenoxy oder Thiophenyl substituiert sind, die ihrerseits durch Halogen, Trifluormethyl oder Trifluormethoxy substituiert sein können,
oder die Cyclen gegebenenfalls durch eine Gruppe der Formel -NR⁹R¹⁰ substituiert sind,
worin
R⁹ und R¹⁰ gleich oder verschieden sind und die oben angegebene Bedeutung von R³ und R⁴ haben,
X geradkettiges oder verzweigtes Alkylen oder Alkenylen mit jeweils bis zu 8 Kohlenstoffatomen bedeutet, die gegebenenfalls bis zu 2-fach durch Hydroxy substituiert sind,
R⁷ Wasserstoff oder Halogen bedeutet,
und
R⁸ Wasserstoff, Halogen, Azido, Trifluormethyl, Hydroxy, Trifluormethoxy, geradkettiges oder verzweigtes Alkoxy mit bis zu 5 Kohlenstoffatomen oder einen Rest der Formel -NR¹¹R¹² bedeutet,
worin
R¹¹ und R¹² gleich oder verschieden sind und die oben angegebene Bedeutung von R³ und R⁴ haben,
oder
R⁷ und R⁸ gemeinsam mit dem C-Atom eine Carbonylgruppe bilden,
E für Cycloalkyl mit 3 bis 8 Kohlenstoffatomen steht, oder für geradkettiges oder verzweigtes Alkyl mit bis zu 8 Kohlenstoffatomen steht, das gegebenenfalls durch Cycloalkyl mit 3 bis 8 Kohlenstoffatomen oder durch Hydroxy substituiert ist,
R¹ und R² gemeinsam eine Alkylenkette mit bis zu 6 Kohlenstoffatomen bilden, die durch ein Sauerstoff- oder Schwefelatom oder durch die Gruppe -SO₂- oder -NR¹³ unterbrochen ist,
wobei
R¹³ Wasserstoff oder geradkettiges oder verzweigtes Alkyl oder Acyl jeweils mit bis zu 6 Kohlenstoffatomen bedeutet, oder Benzyl oder Phenyl bedeutet, die gegebenenfalls bis zu 2-fach gleich oder verschieden durch Halogen, Hydroxy, Nitro, Trifluormethyl oder geradkettiges oder verzweigtes Alkyl oder Acyl mit jeweils bis zu 6 Kohlenstoffatomen substituiert sind,
und wobei der so gebildete heterocyclische Ring, der auch benzokondensiert sein kann und eine Doppelbindung enthalten kann, stets durch eine Oxogruppe oder einen Rest der Formel 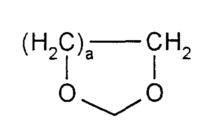 oder -OR¹⁴ substituiert sein muß,
worin
a eine Zahl 1, 2 oder 3 bedeutet
und
R¹⁴ Wasserstoff oder geradkettiges oder verzweigtes Alkyl, hydroxysubstituiertes Alkyl, Acyl oder Alkoxycarbonyl mit jeweils bis zu 6 Kohlenstoffatomen oder einen Rest der Formel -SiR¹⁵R¹⁶R¹⁷ bedeutet,
worin
R¹⁵, R¹⁶ und R¹⁷ gleich oder verschieden sind und Phenyl, geradkettiges oder verzweigtes Alkyl mit bis zu 6 Kohlenstoffatomen bedeuten,
und der heterocyclische und/oder benzokondensierte Ring (R¹/R²) gegebenenfalls bis zu 5-fach gleich oder verschieden, gegebenenfalls auch geminal, durch geradkettiges oder verzweigtes Alkoxy oder Alkoxycarbonyl mit jeweils bis zu 6 Kohlenstoffatomen, Trifluormethyl, Halogen, Hydroxy, Carbonyl oder Phenyl substituiert ist, das seinerseits durch Halogen, Trifluormethyl, Nitro, Hydroxy oder durch geradkettiges oder verzweigtes Alkyl, Alkoxy oder Alkoxycarbonyl mit jeweils bis zu 6 Kohlenstoffatomen substituiert sein kann,
und/oder gegebenenfalls bis zu 6-fach, gegebenenfalls auch geminal, gleich oder verschieden durch Cycloalkyl- oder Cycloalkyloxy mit jeweils 3 bis 8 Kohlenstoffatomen oder durch geradkettiges oder verzweigtes Alkyl mit bis zu 6 Kohlenstoffatomen substituiert ist, das seinerseits durch Hydroxy, Trifluormethyl, Phenyl oder durch geradkettiges oder verzweigtes Alkoxy mit bis zu 5 Kohlenstoffatomen substituiert sein kann,
und/oder gegebenenfalls durch einen spiro-verknüpften Rest der Formel 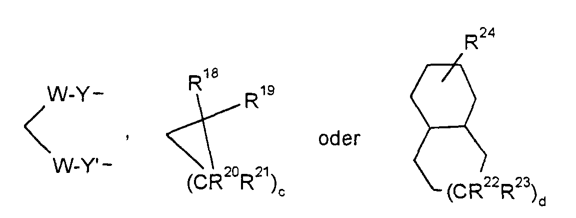 substituiert ist,
worin
W entweder ein Sauerstoff oder ein Schwefelatom bedeutet,
Y und Y' gemeinsam eine 2 bis 6-gliedrige geradkettige oder verzweigte Alkylenkette bilden
c eine Zahl 1, 2, 3, 4, 5, 6 oder 7 bedeutet,
d eine Zahl 1 oder 2 bedeutet,
R¹⁸, R¹⁹, R²⁰, R²¹, R²², R²³ und R²⁴ gleich oder verschieden sind und Wasserstoff, Trifluormthyl, Phenyl, Halogen oder geradkettiges oder verzweigtes Alkyl oder Alkoxy mit jeweils bis zu 6 Kohlenstoffatomen bedeuten oder
R¹⁸ und R¹⁹ oder R²⁰ und R²¹ jeweils gemeinsam eine geradkettige oder verzweigte Alkylenkette mit bis zu 6 Kohlenstoffatomen bilden oder
R¹⁸ und R¹⁹ oder R²⁰ und R²¹ jeweils gemeinsam einen Rest der Formel 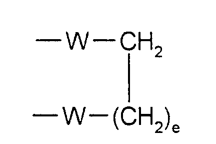 bilden, worin
W die oben angegebene Bedeutung hat,
e eine Zahl 1, 2, 3, 4, 5 , 6 oder 7 bedeutet
und deren Salze und N-oxide.

2. Heterocyclisch kondensierte Pyridine der Formel nach Anspruch 1
in welcher
A für Naphthyl oder Phenyl steht, die gegebenenfalls bis zu 3-fach gleich oder verschieden durch Fluor, Chlor, Brom, Hydroxy, Trifluormethyl, Trifluormethoxy, Nitro oder durch geradkettiges oder verzweigtes Alkyl, Acyl oder Alkoxy mit jeweils bis zu 6 Kohlenstoffatomen oder durch eine Gruppe der Formel -NR³R⁴ substituiert sind,
worin
R³ und R⁴ gleich oder verschieden sind und Wasserstoff, Phenyl oder geradkettiges oder verzweigtes Alkyl mit bis zu 4 Kohlenstoffatomen bedeuten,
D für einen Rest der Formel
R⁵―X―
oder 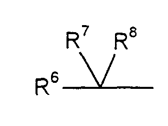 steht,
worin
R⁵ und R⁶ unabhängig voneinander Cyclopropyl, Cyclopentyl oder Cyclohexyl bedeuten, oder Naphthyl, Phenyl, Pyridyl, Chinolyl, Indolyl, Benzthiazolyl oder Tetrahydronaphthalinyl bedeuten, die gegebenenfalls bis zu 3-fach gleich oder verschieden durch Trifluormethyl, Trifluormethoxy, Fluor, Chlor, Brom, Hydroxy, Carboxyl, durch geradkettiges oder verzweigtes Alkyl, Acyl, Alkoxy oder Alkoxycarbonyl mit jeweils bis zu 5 Kohlenstoffatomen oder durch Phenyl, Phenoxy oder Thiophenyl substituiert sind, die ihrerseits durch Fluor, Chlor, Brom, Trifluormethyl oder Trifluormethoxy substituiert sein können,
oder die Cyclen gegebenenfalls durch eine Gruppe der Formel -NR⁹R¹⁰ substituiert sind,
worin
R⁹ und R¹⁰ gleich oder verschieden sind und die oben angegebene Bedeutung von R³ und R⁴ haben,
X geradkettiges oder verzweigtes Alkylen oder Alkenylen mit jeweils bis zu 6 Kohlenstoffatomen bedeutet, die gegebenenfalls bis zu 2-fach durch Hydroxy substituiert sind,
R⁷ Wasserstoff, Fluor oder Chlor bedeutet,
und
R⁸ Wasserstoff, Fluor, Chlor, Brom, Azido, Trifluormethyl, Hydroxy, Trifluormethoxy, geradkettiges oder verzweigtes Alkoxy mit bis zu 4 Kohlenstoffatomen oder einen Rest der Formel -NR¹¹R¹² bedeutet,
worin
R¹¹ und R¹² gleich oder verschieden sind und die oben angegebene Bedeutung von R³ und R⁴ haben,
oder
R⁷ und R⁸ gemeinsam mit dem C-Atom eine Carbonylgruppe bilden,
E für Cyclopropyl, Cyclobutyl, Cyclopentyl, Cyclohexyl oder Cycloheptyl steht, oder
für geradkettiges oder verzweigtes Alkyl mit bis zu 6 Kohlenstoffatomen steht, das gegebenenfalls durch Cyclopropyl, Cyclobutyl, Cyclopentyl, Cyclohexyl, Cycloheptyl oder durch Hydroxy substituiert ist,
R¹ und R² gemeinsam eine Alkylenkette mit bis zu 5 Kohlenstoffatomen bilden, die durch ein Sauerstoff- oder Schwefelatom oder einen durch die Gruppe -SO₂- oder -NR¹³ unterbrochen ist, wobei
R¹³ Wasserstoff oder geradkettiges oder verzweiges Alkyl oder Acyl mit jeweils bis zu 5 Kohlenstoffatomen bedeutet, oder Benzyl oder Phenyl bedeutet, die gegebenenfalls bis zu 2-fach gleich oder verschieden durch Fluor, Chlor, Brom, Hydroxy, Nitro, Trifluormethyl oder geradkettiges oder verzweigtes Alkyl oder Acyl mit jeweils bis zu 4 Kohlenstoffatomen substituiert sind,
und wobei der so gebildete heterocyclische Ring, der auch benzokondensiert sein kann und eine Doppelbindung enthalten kann, stets durch einen Rest der Formel 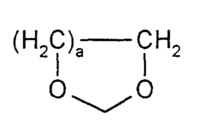 oder -OR¹⁴ substituiert sein muß,
worin
a eine Zahl 1, 2 oder 3 bedeutet
und
R¹⁴ Wasserstoff oder geradkettiges oder verzweigtes Alkyl, hydroxysubstituiertes Alkyl oder Alkoxycarbonyl mit jeweils bis zu 5 Kohlenstoffatomen oder einen Rest der Formel -SiR¹⁵R¹⁶R¹⁷ bedeutet,
worin
R¹⁵, R¹⁶ und R¹⁷ gleich oder verschieden sind und Phenyl, geradkettiges oder verzweigtes Alkyl mit bis zu 5 Kohlenstoffatomen bedeuten,
und/oder gegebenenfalls bis zu 4-fach, gegebenenfalls auch geminal, gleich oder verschieden durch Cyclopropyl, Cyclopropyloxy, Cyclopentyl, Cyclopentyloxy, Cyclohexyl, Cyclohexyloxy oder durch geradkettiges oder verzweigtes Alkyl mit bis zu 4 Kohlenstoffatomen substituiert ist, das seinerseits durch Hydroxy, Trifluormethyl, Phenyl oder durch geradkettiges oder verzweigtes Alkoxy mit bis zu 3 Kohlenstoffatomen substituiert sein kann
und/oder gegebenenfalls durch einen spiro-verknüpften Rest der Formel 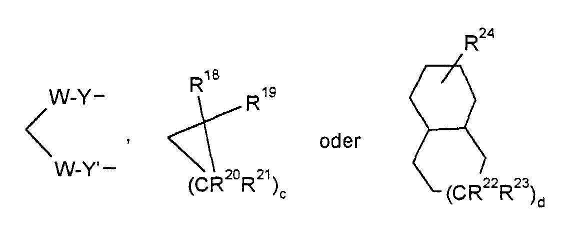
substituiert ist,
worin
W entweder ein Sauerstoff oder ein Schwefelatom bedeutet,
Y und Y' gemeinsam eine 2- bis 5-gliedrige, geradkettige oder verzweigte Alkylenkette bilden
c eine Zahl 1, 2, 3, 4, 5 oder 6 bedeutet,
d eine Zahl 1 oder 2 bedeutet,
R¹⁸, R¹⁹, R²⁰, R²¹, R²², R²³ und R²⁴ gleich oder verschieden sind und Wasserstoff, Trifluormethyl, Phenyl, Fluor, Chlor, Brom oder geradkettiges oder verzweigtes Alkyl oder Alkoxy mit jeweils bis zu 5 Kohlenstoffatomen bedeuten oder
R¹⁸ und R¹⁹ oder R²⁰ und R²¹ jeweils gemeinsam eine geradkettige
oder verzweigte Alkylenkette mit bis zu 5 Kohlenstoffatomen bilden oder
R¹⁸ und R¹⁹ oder R²⁰ und R²¹ jeweils gemeinsam einen Rest der Formel 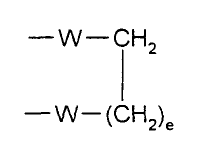 bilden, worin
W die oben angegebene Bedeutung hat,
e eine Zahl 1, 2, 3, 4, 5 oder 6 bedeutet,
und deren Salze und N-oxide.

3. Heterocyclisch kondensierte Pyridine der Formel nach Anspruch 1
in welcher
A für Phenyl steht, das gegebenenfalls durch Fluor, Chlor, Brom, Hydroxy, Trifluormethyl, Trifluprmethoxy, Nitro oder durch geradkettiges oder verzweigtes Alkyl oder Alkoxy mit jeweils bis zu 3 Kohlenstoffatomen substituiert ist,
D für einen Rest der Formel
R⁵―X―
oder 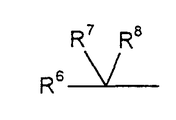 steht,
worin
R⁵ und R⁶ unabhängig voneinander Cyclopropyl, Naphthyl, Phenyl, Pyridyl, Chinolyl, Indolyl, Benzthiazolyl oder Tetrahydronaphthalinyl bedeuten, die gegebenenfalls bis zu 2-fach gleich oder verschieden durch Trifluormethyl, Trifluormethoxy, Fluor, Chlor, Brom, Hydroxy, Carboxyl, Amino, durch geradkettiges oder verzweigtes Alkyl, Acyl, Alkoxy oder Alkoxycarbonyl mit jeweils bis zu 4 Kohlenstoffatomen oder durch Phenyl, Phenoxy oder Thiophenyl substituiert sind, die ihrerseits durch Fluor, Chlor, Brom, Trifluormethyl oder Trifluormethoxy substituiert sein können,
X geradkettiges oder verzweigtes Alkyl oder Alkenyl mit bis zu 4 Kohlenstoffatomen bedeutet, die gegebenenfalls durch Hydroxy substituiert ist,
R⁷ Wasserstoff oder Fluor bedeutet,
und
R⁸ Wasserstoff, Fluor, Chlor, Brom, Azido, Trifluormethyl, Hydroxy, Amino, Trifluormethoxy oder Methoxy bedeutet,
oder
R⁷ und R⁸ gemeinsam mit dem Kohlenstoffatom eine Carbonylgruppe bilden,
E für Cyclopropyl, Cyclobutyl, Cyclopentyl, Cyclohexyl oder Cycloheptyl steht, oder
für geradkettiges oder verzweigtes Alkyl mit bis zu 5 Kohlenstoffatomen steht, das gegebenenfalls durch Cyclopentyl oder Cyclohexyl substituiert ist,
R¹ und R² gemeinsam eine Alkylenkette mit bis zu 4 Kohlenstoffatomen bilden, die durchein Sauerstoff- oder Schwefelatom oder die Gruppe -SO₂- oder -NR¹³ unterbrochen ist,
wobei
R¹³ Wasserstoff oder geradkettiges oder verzweigtes Alkyl oder Acyl mit jeweils bis zu 4 Kohlenstoffatomen bedeutet, oder Benzyl oder Phenyl bedeutet, die gegebenenfalls bis zu 2-fach gleich oder verschieden durch Fluor, Chlor, Brom, Hydroxy, Nitro, Trifluormethyl oder geradkettiges oder verzweigtes Alkyl oder Acyl mit jeweils bis zu 3 Kohlenstoffatomen substituiert sind,
und wobei der so gebildete heterocyclische Ring, der auch benzokondensiert sein kann, und der eine Doppelbindung enthalten kann, stets durch einen Rest der Formel 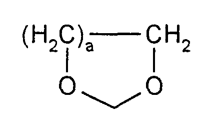 oder -OR¹⁴ substituiert sein muß,
worin
a eine Zahl 1, 2 oder 3 bedeutet
und
R¹⁴ Wasserstoff oder geradkettiges oder verzweigtes Alkyl, hydroxysubstituiertes Alkyl oder Alkoxycarbonyl mit jeweils bis zu 4 Kohlenstoffatomen oder einen Rest der Formel -SiR¹⁵R¹⁶R¹⁷ bedeutet,
worin
R¹⁵, R¹⁶ und R¹⁷ gleich oder verschieden sind und Phenyl, geradkettiges oder verzweigtes Alkyl mit bis zu 4 Kohlenstoffatomen bedeuten,
und der heterocyclische und/oder benzokondensierte Ring (R¹/R²) gegebenenfalls bis zu 3-fach gleich oder verschieden, gegebenenfalls auch geminal, durch geradkettiges oder verzweigtes Alkoxy oder Alkoxycarbonyl mit jeweils bis zu 3 Kohlenstoffatomen, Hydroxy, Carboxyl oder Phenyl substituiert sind, das seinerseits durch Fluor, Chlor, Brom, Trifluormethyl oder durch geradkettiges oder verzweigtes Alkyl mit jeweils bis zu 3 Kohlenstoffatome substituiert sein kann,
und/oder gegebenenfalls bis zu 3-fach, gegebenenfalls auch geminal, gleich oder verschieden durch Cyclopropyl, Cyclopropyloxy, Cyclopentyl, Cyclopentyloxy, Cyclohexyl, Cyclohexyloxy oder durch geradkettiges oder verzweigtes Alkyl mit bis zu 3 Kohlenstoffatomen substituiert ist, das seinerseits durch Hydroxy, Trifluormethyl, Phenyl oder Methoxy substituiert sein kann
und/oder gegebenenfalls durch einen spiro-verknüpften Rest der Formel 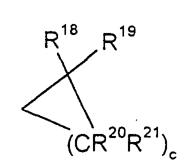 substituiert ist,
worin
c eine Zahl 1, 2, 3, 4 oder 5 bedeutet,
R¹⁸, R¹⁹, R²⁰ und R²¹ gleich oder verschieden sind und Wasserstoff, Trifluormethyl, Phenyl, Fluor, Chlor, Brom oder geradkettiges oder verzweigtes Alkyl oder Alkoxy mit jeweils bis zu 4 Kohlenstoffatomen bedeuten
oder
R¹⁸ und R¹⁹ oder R²⁰ und R²¹ jeweils gemeinsam eine geradkettige oder verzweigte Alkylenkette mit bis zu 4 Kohlenstoffatomen bilden
oder
R¹⁸ und R¹⁹ oder R²⁰ und R²¹ jeweils gemeinsam einen Rest der Formel 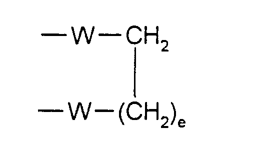 bilden,
worin
W entweder ein Sauerstoff- oder Schwefelatom bedeutet und
e eine Zahl 1, 2, 3, 4 oder 5 bedeutet,
und deren Salze und N-Oxide.

4. Heterocyclisch kondensierte Pyridine der Formel nach Anspruch 1
in welcher
A für Phenyl steht, das gegebenenfalls durch Fluor, Chlor oder Brom substituiert ist
und
E für Cyclopentyl oder Isopropyl steht.

5. Heterocyclisch kondensierte Pyridine nach Anspruch 1 bis 4 als Arzneimittel.

6. Verfahren zur Herstellung von heterocyclisch kondensierten Pyridinen nach Anspruch 1 bis 4 **dadurch gekennzeichnet, daß** man in die
Verbindungen der allgemeinen Formel (II) 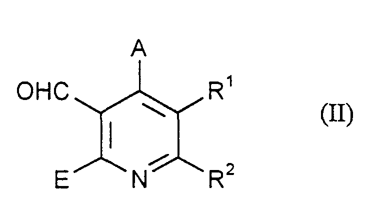 in welcher
A, E, R¹ und R² die oben angegebene Bedeutung haben,
zunächst mit metallorganischen Reagenzien im Sinne einer Grignard- oder Wittig-Reaktion den Substituenten D in inerten Lösemitteln einführt,
und gegebenenfalls die unter A, E und/oder R¹ und R² aufgeführten Substituenten nach üblichen Methoden, variiert oder einführt.

7. Arzneimittel, enthaltend mindestens ein heterocyclisch kondensiertes Pyridin nach Ansprüchen 1 bis 4 sowie ein physiologisch unbedenkliches Formulierungshilfsmittel.

8. Arzneimittel nach Anspruch 7 zur Behandlung von Hyperlipoproteinaemia und Arteriosklerose.

9. Verwendung von heterocyclisch kondensierten Pyridinen nach Ansprüchen 1 bis 4 zur Herstellung von Arzneimitteln.

10. Verwendung nach Anspruch 9 zur Herstellung von Arzneimitteln zur Behandlung von Ateriosklerose.

## Claims

1. Heterocyclic-fused pyridines of the general formula (I) 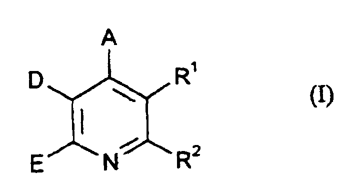 in which
A represents aryl having 6 to 10 carbon atoms, which is optionally substituted up to 5 times in an identical or different manner by halogen, hydroxyl, trifluoromethyl, trifluoromethoxy, nitro or by straight-chain or branched alkyl, acyl, hydroxyalkyl or alkoxy each having up to 7 carbon atoms, or by a group of the formula -NR³R⁴,
in which
R³ and R⁴ are identical or different and denote hydrogen, phenyl or straight-chain or branched alkyl having up to 6 carbon atoms,
D represents a radical of the formula
R⁵-X-
or 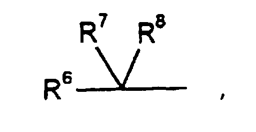 in which
R⁵ and R⁶ independently of one another denote cycloalkyl having 3 to 8 carbon atoms, or
aryl having 6 to 10 carbon atoms,
or a 5- to 7-membered aromatic, optionally benzo-fused heterocycle having up to 3 heteroatoms from the series consisting of S, N and/or O, each of which is optionally substituted up to 5 times in an identical or different manner by trifluoromethyl, nitro, trifluoromethoxy, halogen, hydroxyl, carboxyl, by straight-chain or branched alkyl, acyl, alkoxy or alkoxycarbonyl each having up to 6 carbon atoms or by phenyl, phenoxy or thiophenyl, which for their part can be substituted by halogen, trifluoromethyl or trifluoromethoxy,
or the cycles are optionally substituted by a group of the formula -NR⁹R¹⁰,
in which
R⁹ and R¹⁰ are identical or different and have the meaning of R³ and R⁴ indicated above,
X denotes straight-chain or branched alkylene or alkenylene each having up to 8 carbon atoms, each of which is optionally substituted up to 2 times by hydroxyl,
R⁷ denotes hydrogen or halogen
and
R⁸ denotes hydrogen, halogen, azido, trifluoromethyl, hydroxyl, trifluoromethoxy, straight-chain or branched alkoxy having up to 5 carbon atoms or a radical of the formula -NR¹¹R¹²,
in which
R¹¹ and R¹² are identical or different and have the meaning of R³ and R⁴ indicated above,
or
R⁷ and R⁸, together with the C atom, form a carbonyl group,
E represents cycloalkyl having 3 to 8 carbon atoms, or
represents straight-chain or branched alkyl having up to 8 carbon atoms, which is optionally substituted by cycloalkyl having 3 to 8 carbon atoms or by hydroxyl,
R¹ and R² together form an alkylene chain having up to 6
carbon atoms, which is interrupted by an oxygen or sulphur atom or by the group -SO₂- or -NR¹³,
where
R¹³ denotes hydrogen or straight-chain or branched alkyl or acyl each having up to 6 carbon atoms, or
denotes benzyl or phenyl, each of which is optionally substituted up to 2 times in an identical or different manner by halogen, hydroxyl, nitro, trifluoromethyl or straight-chain or branched alkyl or acyl each having up to 6 carbon atoms,
and where the heterocyclic ring thus formed, which can also be benzo-fused and can contain a double bond, must always be substituted by an oxo group or a radical of the formula 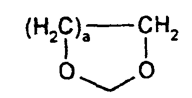 or -OR¹⁴,
in which
a denotes a number 1, 2 or 3
and
R¹⁴ denotes hydrogen or straight-chain or branched alkyl, hydroxy-substituted alkyl, acyl or alkoxycarbonyl each having up to 6 carbon atoms or a radical of the formula -SiR¹⁵R¹⁶R¹⁷,
in which
R¹⁵, R¹⁶ and R¹⁷ are identical or different and denote phenyl, straight-chain or branched alkyl having up to 6 carbon atoms,
and the heterocyclic and/or benzo-fused ring (R¹/R²) is optionally substituted up to 5 times in an identical or different manner, optionally also geminally, by straight-chain or branched alkoxy or alkoxycarbonyl each having up to 6 carbon atoms, trifluoromethyl, halogen, hydroxyl, carbonyl or phenyl which, for its part, can be substituted by halogen, trifluoromethyl, nitro, hydroxyl or by straight-chain or branched alkyl, alkoxy or alkoxycarbonyl each having up to 6 carbon atoms,
and/or is optionally substituted up to 6 times, optionally also geminally, in an identical or different manner by cycloalkyl or cycloalkyloxy each having 3 to 8 carbon atoms or by straight-chain or branched alkyl having up to 6 carbon atoms, which for its part can be substituted by hydroxyl, trifluoromethyl, phenyl or by straight-chain or branched alkoxy having up to 5 carbon atoms,
and/or is optionally substituted by a spiro-linked radical of the formula 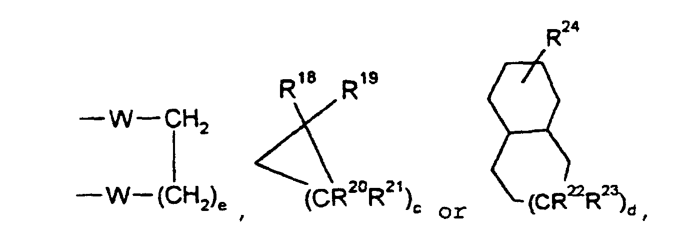 in which
W denotes either an oxygen or a sulphur atom,
Y and Y' together form a 2- to 6-membered straight-chain or branched alkylene chain,
c denotes a number 1, 2, 3, 4, 5, 6 or 7,
d denotes a number 1 or 2,
R¹⁸, R¹⁹, R²⁰, R²¹, R²², R²³ and R²⁴ are identical or different and denote hydrogen, trifluoromethyl, phenyl, halogen or straight-chain or branched alkyl or alkoxy each having up to 6 carbon atoms or
R¹⁸ and R¹⁹ or R²⁰ and R²¹ in each case together form a straight-chain or branched alkylene chain having up to 6 carbon atoms or
R¹⁸ and R¹⁹ or R²⁰ and R²¹ in each case together form a radical of the formula 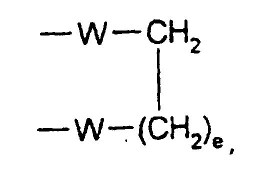 in which
W has the meaning indicated above,
e denotes a number 1, 2, 3, 4, 5, 6 or 7,
and their salts and N-oxides.

2. Heterocyclic-fused pyridines of the formula according to Claim 1
in which
A represents naphthyl or phenyl, each of which is optionally substituted up to 3 times in an identical or different manner by fluorine, chlorine, bromine, hydroxyl, trifluoromethyl, trifluoromethoxy, nitro or by straight-chain or branched alkyl, acyl or alkoxy each having up to 6 carbon atoms or by a group of the formula -NR³R⁴,
in which
R³ and R⁴ are identical or different and denote hydrogen, phenyl or straight-chain or branched alkyl having up to 4 carbon atoms,
D represents a radical of the formula
R⁵-X- or 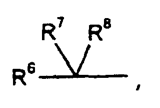 in which
R⁵ and R⁶ independently of one another
denote cyclopropyl, cyclopentyl or cyclohexyl, or
denote naphthyl, phenyl, pyridyl, quinolyl, indolyl, benzothiazolyl or tetrahydronaphthalenyl, each of which is optionally substituted up to 3 times in an identical or different manner by trifluoromethyl, trifluoromethoxy, fluorine, chlorine, bromine, hydroxyl, carboxyl, by straight-chain or branched alkyl, acyl, alkoxy or alkoxycarbonyl each having up to 5 carbon atoms or by phenyl, phenoxy or thiophenyl, which for their part can be substituted by fluorine, chlorine, bromine, trifluoromethyl or trifluoromethoxy,
or the cycles are optionally substituted by a group of the formula -NR⁹R¹⁰,
in which
R⁹ and R¹⁰ are identical or different and have the meaning of R³ and R⁴ indicated above,
X denotes straight-chain or branched alkylene or alkenylene each having up to 6 carbon atoms, each of which is optionally substituted up to 2 times by hydroxyl,
R⁷ denotes hydrogen, fluorine or chlorine,
and
R⁸ denotes hydrogen, fluorine, chlorine, bromine, azido, trifluoromethyl, hydroxyl, trifluoromethoxy, straight-chain or branched alkoxy having up to 4 carbon atoms or a radical of the formula -NR¹¹R¹²,
in which
R¹¹ and R¹² are identical or different and have the meaning of R³ and R⁴ indicated above,
or
R⁷ and R⁸ together with the C atom form a carbonyl group,
E represents cyclopropyl, cyclobutyl, cyclopentyl, cyclohexyl or cycloheptyl, or
represents straight-chain or branched alkyl having up to 6 carbon atoms, which is optionally substituted by cyclopropyl, cyclobutyl, cyclopentyl, cyclohexyl, cycloheptyl or by hydroxyl,
R¹ and R² together form an alkylene chain having up to 5
carbon atoms, which is interrupted by an oxygen or sulphur atom or by the group -SO₂- or -NR¹³, where
R¹³ denotes hydrogen or straight-chain or branched alkyl or acyl each having up to 5 carbon atoms, or
denotes benzyl or phenyl, each of which is optionally substituted up to 2 times in an identical or different manner by fluorine, chlorine, bromine, hydroxyl, nitro, trifluoromethyl or straight-chain or branched alkyl or acyl each having up to 4 carbon atoms,
and where the heterocyclic ring thus formed, which can also be benzo-fused and can contain a double bond, must always be substituted by a radical of the formula 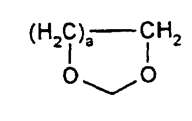 or -OR¹⁴,
in which
a denotes a number 1, 2 or 3
and
R¹⁴ denotes hydrogen or straight-chain or branched alkyl, hydroxy- substituted alkyl or alkoxycarbonyl each having up to 5 carbon atoms or a radical of the formula -SiR¹⁵R¹⁶R¹⁷,
in which
R¹⁵, R¹⁶ and R¹⁷ are identical or different and denote phenyl, straight-chain or branched alkyl having up to 5 carbon atoms,
and the heterocyclic and/or benzo-fused ring (R¹/R²) is optionally substituted up to 3 times in an identical or different manner, optionally also geminally, by straight-chain or branched alkoxy or alkoxycarbonyl each having up to 4 carbon atoms, hydroxyl, carboxyl or phenyl, which for its part can be substituted by fluorine, chlorine, bromine, trifluoromethyl or by straight-chain or branched alkyl or alkoxy each having up to 4 carbon atoms,
and/or is optionally substituted up to 4 times, optionally also geminally, in an identical or different manner by cyclopropyl, cyclopropyloxy, cyclopentyl, cyclopentyloxy, cyclohexyl, cyclohexyloxy or by straight-chain or branched alkyl having up to 4 carbon atoms, which for its part can be substituted by hydroxyl, trifluoromethyl, phenyl or by straight-chain or branched alkoxy having up to 3 carbon atoms
and/or is optionally substituted by a spiro-linked radical of the formula 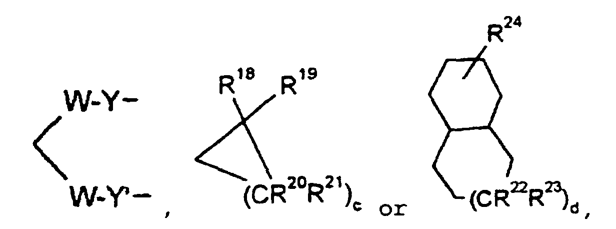 in which
W denotes either an oxygen or a sulphur atom,
Y and Y' together form a 2- to 5-membered, straight-chain or branched alkylene chain,
c denotes a number 1, 2, 3, 4, 5 or 6,
d denotes a number 1 or 2,
R¹⁸, R¹⁹, R²⁰, R²¹, R²², R²³ and R²⁴ are identical or different and denote hydrogen, trifluoromethyl, phenyl, fluorine, chlorine, bromine or straight-chain or branched alkyl or alkoxy each having up to 5 carbon atoms or
R¹⁸ and R¹⁹ or R²⁰ and R²¹ in each case together form a straight-chain or branched alkylene chain having up to 5 carbon atoms or
R¹⁸ and R¹⁹ or R²⁰ and R²¹ in each case together form a radical of the formula 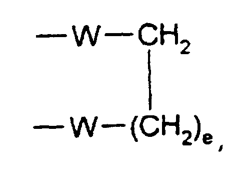 in which
W has the meaning indicated above,
e denotes a number 1, 2, 3, 4, 5 or 6,
and their salts and N-oxides.

3. Heterocyclic-fused pyridines of the formula according to Claim 1
in which
A represents phenyl which is optionally substituted by fluorine, chlorine, bromine, hydroxyl, trifluoromethyl, trifluoromethoxy, nitro or by straight-chain or branched alkyl or alkoxy each having up to 3 carbon atoms,
D represents a radical of the formula
R⁵-X- or 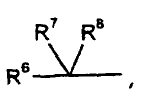 in which
R⁵ and R⁶ independently of one another denote cyclopropyl, naphthyl, phenyl, pyridyl, quinolyl, indolyl, benzothiazolyl or tetrahydronaphthalenyl, each of which is optionally substituted up to 2 times in an identical or different manner by trifluoromethyl, trifluoromethoxy, fluorine, chlorine, bromine, hydroxyl, carboxyl, amino, by straight-chain or branched alkyl, acyl, alkoxy or alkoxycarbonyl each having up to 4 carbon atoms or by phenyl, phenoxy or thiophenyl, which for their part can be substituted by fluorine, chlorine, bromine, trifluoromethyl or trifluoromethoxy,
X denotes straight-chain or branched alkylene or alkenylene having up to 4 carbon atoms, each of which is optionally substituted by hydroxyl,
R⁷ denotes hydrogen or fluorine
and
R⁸ denotes hydrogen, fluorine, chlorine, bromine, azido, trifluoromethyl, hydroxyl, amino, trifluoromethoxy or methoxy,
or
R⁷ and R⁸, together with the carbon atom, form a carbonyl group,
E represents cyclopropyl, cyclobutyl, cyclopentyl, cyclohexyl or cycloheptyl, or represents straight-chain or branched alkyl having up to 5 carbon atoms, which is optionally substituted by cyclopentyl or cyclohexyl,
R¹ and R² together form an alkylene chain having up to 4 carbon atoms, which is interrupted by an oxygen or sulphur atom or by the group -SO₂- or -NR¹³,
where
R¹³ denotes hydrogen or straight-chain or branched alkyl or acyl each having up to 4 carbon atoms, or
denotes benzyl or phenyl, each of which is optionally substituted up to 2 times in an identical or different manner by fluorine, chlorine, bromine, hydroxyl, nitro, trifluoromethyl or straight-chain or branched alkyl or acyl each having up to 3 carbon atoms,
and where the heterocyclic ring thus formed, which can also be benzo-fused and which can contain a double bond, must always be substituted by a radical of the formula 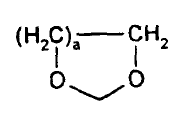 or -OR¹⁴,
in which
a denotes a number 1, 2 or 3
and
R¹⁴ denotes hydrogen or straight-chain or branched alkyl, hydroxy-substituted alkyl or alkoxycarbonyl each having up to 4 carbon atoms or a radical of the formula -SiR¹⁵R¹⁶R¹⁷,
in which
R¹⁵, R¹⁶ and R¹⁷ are identical or different and denote phenyl, straight-chain or branched alkyl having up to 4 carbon atoms,
and the heterocyclic and/or benzo-fused ring (R¹/R²) is optionally substituted up to 3 times in an identical or different manner, optionally also geminally, by straight-chain or branched alkoxy or alkoxycarbonyl each having up to 3 carbon atoms, hydroxyl, carboxyl or phenyl which, for its part, can be substituted by fluorine, chlorine, bromine, trifluoromethyl or by straight-chain or branched alkyl having up to 3 carbon atoms,
and/or is optionally substituted up to 3 times, optionally also geminally, in an identical or different manner by cyclopropyl, cyclopropyloxy, cyclopentyl, cyclopentyloxy, cyclohexyl or by straight-chain or branched alkyl having up to 3 carbon atoms, which for its part can be substituted by hydroxyl, trifluoromethyl, phenyl or methoxy
and/or is optionally substituted by a spiro-linked radical of the formula 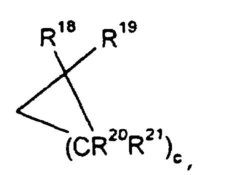 in which
c denotes a number 1, 2, 3, 4 or 5,
R¹⁸, R¹⁹, R²⁰ and R²¹ are identical or different and denote hydrogen, trifluoromethyl, phenyl, fluorine, chlorine, bromine or straight-chain or branched alkyl or alkoxy each having up to 4 carbon atoms
or
R¹⁸ and R¹⁹ or R²⁰ and R²¹ in each case together form a straight-chain or branched alkylene chain having up to 4 carbon atoms
or
R¹⁸ and R¹⁹ or R²⁰ and R²¹ in each case together form a radical of the formula 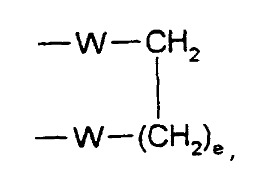 in which
W denotes either an oxygen or sulphur atom and
e denotes a number 1, 2, 3, 4 or 5,
and their salts and N-oxides.

4. Heterocyclic-fused pyridines of the formula according to Claim 1
in which
A represents phenyl which is optionally substituted by fluorine, chlorine or bromine
and
E represents cyclopentyl or isopropyl.

5. Heterocyclic-fused pyridines according to Claims 1 to 4 as medicaments.

6. Process for the preparation of heterocyclic-fused pyridines according to Claims 1 to 4, **characterized in that** in the compounds of the general formula (II) 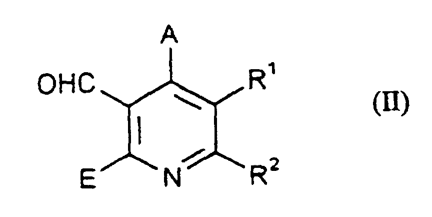 in which
A, E, R¹ and R² have the meaning indicated above,
first, with organometallic reagents, the substituent D is introduced in inert solvents according to a Grignard or Wittig reaction,
and if appropriate the substituents mentioned under A, E and/or R¹ and R² are varied or introduced according to customary methods.

7. Medicaments comprising at least one heterocyclic-fused pyridine according to Claims 1 to 4, and a physiologically acceptable formulation auxiliary.

8. Medicaments according to Claim 7 for the treatment of hyperlipoproteinaemia and asteriosclerosis.

9. Use of heterocyclic-fused pyridines according to Claims 1 to 4 for the production of medicaments.

10. Use according to Claim 9 for the production of medicaments for the treatment of arteriosclerosis.

## Revendications

1. Pyridines condensées à un hétérocycle, de formule générale (I) 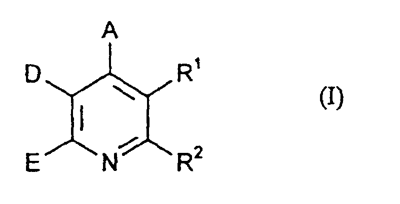 dans laquelle
A est un reste aryle ayant 6 à 10 atomes de carbone, qui est éventuellement substitué jusqu'à 5 fois identiques ou différentes par un halogène, un groupe hydroxy, trifluorométhyle, trifluorométhoxy, nitro ou par un groupe alkyle, acyle, hydroxyalkyle ou alkoxy linéaire ou ramifié ayant chacun jusqu'à 7 atomes de carbone, ou par un groupe de formule -NR³R⁴,
où
R³ et R⁴ sont identiques ou différents et représentent l'hydrogène, un groupe phényle ou un groupe alkyle linéaire ou ramifié ayant jusqu'à 6 atomes de carbone,
D est un reste de formule
R⁵―X―
ou 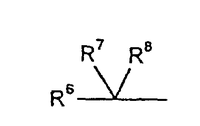 dans laquelle
R⁵ et R⁶ représentent, indépendamment l'un de l'autre, un groupe cycloalkyle ayant 3 à 8 atomes de carbone, ou
un groupe aryle ayant 6 à 10 atomes de carbone,
ou un hétérocycle aromatique pentagonal à heptagonal éventuellement condensé au benzène, ayant jusqu'à 3 hétéroatomes de la série S, N et/ou O, qui sont substitués, le cas échéant, jusqu'à 5 fois identiques ou différentes par un groupe trifluorométhyle, nitro, trifluorométhoxy, un halogène, un groupe hydroxy, un groupe carboxyle, par un groupe alkyle, acyle, alkoxy ou alkoxycarbonyle linéaire ou ramifié ayant chacun jusqu'à 6 atomes de carbone, ou par un groupe phényle, un groupe phénoxy ou un groupe thiophényle qui peuvent eux-mêmes être substitués par un halogène, un groupe trifluorométhyle ou trifluorométhoxy,
ou bien les cycles sont éventuellement substitués par un groupe de formule -NR⁹R¹⁰
dans laquelle
R⁹ et R¹⁰ sont identiques ou différents et ont la définition indiquée ci-dessus pour R³ et R⁴,
X est un groupe alkylène ou un groupe alcénylène linéaire ou ramifié ayant chacun jusqu'à 8 atomes de carbone, qui sont éventuellement substitués jusqu'à 2 fois par un radical hydroxy,
R⁷ est l'hydrogène ou un halogène,
et
R⁸ est l'hydrogène, un halogène, un groupe azido, trifluorométhyle, hydroxy, trifluorométhoxy, un groupe alkoxy linéaire ou ramifié ayant jusqu'à 5 atomes de carbone ou un reste de formule -NR¹¹R¹², dans laquelle
R¹¹ et R¹² sont identiques ou différents et ont la définition indiquée ci-dessus pour R³ et R⁴,
ou bien
R⁷ et R⁸ forment un groupe carbonyle conjointement avec l'atome de carbone,
E est un reste cycloalkyle ayant 3 à 8 atomes de carbone ou un reste alkyle linéaire ou ramifié ayant jusqu'à 8 atomes de carbone, qui porte éventuellement un substituant cycloalkyle ayant 3 à 8 atomes de carbone ou un substituant hydroxy,
R¹ et R² forment ensemble une chaîne alkylénique ayant jusqu'à 6 atomes de carbone, qui est interrompue par un atome d'oxygène ou de soufre ou par le groupe -SO₂- ou -NR¹³-,
où
R¹³ représente l'hydrogène ou un reste alkyle ou acyle linéaire ou ramifié ayant chacun jusqu'à 6 atomes de carbone ou bien un reste benzyle ou un reste phényle qui sont éventuellement substitués jusqu'à 2 fois identiques ou différentes par un halogène, un groupe hydroxy, nitro, trifluorométhyle ou par un groupe alkyle ou acyle linéaire ou ramifié ayant chacun jusqu'à 6 atomes de carbone,
et le noyau hétérocyclique ainsi formé, qui peut aussi être condensé au benzène et peut contenir une double liaison, doit constamment être substitué par un groupe oxo ou par un reste de formule 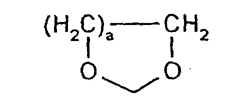 ou -OR¹⁴,
où
a désigne le nombre 1, 2 ou 3
et
R¹⁴ est l'hydrogène ou un reste alkyle, alkyle à substituant hydroxy, acyle ou alkoxycarbonyle linéaire ou ramifié ayant chacun jusqu'à 6 atomes de carbone, ou un reste de formule -SiR¹⁵R¹⁶R¹⁷,
dans laquelle
R¹⁵, R¹⁶ et R¹⁷ sont identiques ou différents et représentent un groupe phényle, alkyle linéaire ou ramifié ayant jusqu'à 6 atomes de carbone,
et le noyau (R¹/R²) hétérocyclique et/ou condensé au benzène porte, le cas échéant, jusqu'à 5 substituants identiques ou différents, éventuellement géminés, alkoxy ou alkoxycarbonyle linéaire ou ramifié ayant chacun jusqu'à 6 atomes de carbone, trifluorométhyle, halogéno, hydroxy, carbonyle ou phényle, qui peut lui-même porter un substituant halogéno, trifluorométhyle, nitro, hydroxy ou un substituant alkyle, alkoxy ou alkoxycarbonyle linéaire ou ramifié ayant chacun jusqu'à 6 atomes de carbone,
et/ou porte, le cas échéant, jusqu'à 6 substituants identiques ou différents, éventuellement géminés, cycloalkyle ou cycloalkyloxy ayant chacun 3 à 8 atomes de carbone, ou alkyle linéaire ou ramifié ayant jusqu'à 6 atomes de carbone, qui peut lui-même porter un substituant hydroxy, trifluorométhyle, phényle ou un substituant alkoxy linéaire ou ramifié ayant jusqu'à 5 atomes de carbone,
et/ou porte comme substituant un reste à liaison spiro, de formule 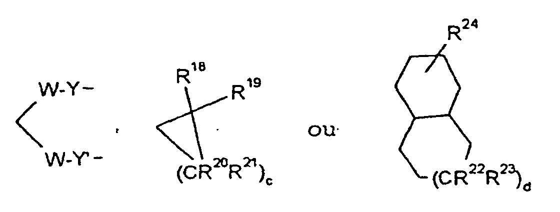 dans laquelle
W désigne un atome d'oxygène ou un atome de soufre,
Y et Y' forment conjointement une chaîne alkylénique linéaire ou ramifiée de 2 à 6 chaînons,
c désigne le nombre 1, 2, 3, 4, 5, 6 ou 7,
d désigne le nombre 1 ou 2,
R¹⁸, R¹⁹, R²⁰, R²¹, R²², R²³ et R²⁴ sont identiques ou différents et représentent l'hydrogène, un reste trifluorométhyle, phényle, un halogène ou un reste alkyle ou alkoxy linéaire ou ramifié ayant chacun jusqu'à 6 atomes de carbone, ou bien
R¹⁸ et R¹⁹ ou R²⁰ et R²¹ forment ensemble une chaîne alkylénique linéaire ou ramifiée ayant jusqu'à 6 atomes de carbone, ou bien
R¹⁸ et R¹⁹ ou R²⁰ et R²¹ forment ensemble dans chaque cas un reste de formule 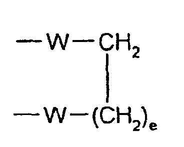
dans laquelle
W a la définition indiquée ci-dessus,
e est le nombre 1, 2, 3, 4, 5, 6 ou 7
et leurs sels et N-oxydes.

2. Pyridines condensées à un hétérocycle, de formule suivant la revendication 1
dans laquelle
A est un reste naphtyle ou un reste phényle, qui est substitué, le cas échéant, jusqu'à 3 fois identiques ou différentes par du fluor, du chlore, du brome, un groupe hydroxy, trifluorométhyle, trifluorométhoxy, nitro ou par un groupe alkyle, acyle ou alkoxy linéaire ou ramifié ayant dans chaque cas jusqu'à 6 atomes de carbone ou par un groupe de formule -NR³R⁴,
dans laquelle
R³ et R⁴ sont identiques ou différents et représentent l'hydrogène, un groupe phényle ou un groupe alkyle linéaire ou ramifié ayant jusqu'à 4 atomes de carbone,
D désigne un reste de formule
R⁵―X―
ou 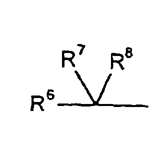 steht,
dans laquelle
R⁵ et R⁶ représentent, indépendamment l'un de l'autre, un groupe cyclopropyle, cyclopentyle ou cyclohexyle ou un groupe naphtyle, phényle, pyridyle, quinolyle, indolyle, benzothiazolyle ou tétrahydronaphtalényle, qui sont substitués, le cas échéant, jusqu'à 3 fois identiques ou différentes par un groupe trifluorométhyle, trifluorométhoxy, le fluor, le chlore, le brome, un groupe hydroxy, carboxyle, par un groupe alkyle, acyle, alkoxy ou alkoxycarbonyle linéaire ou ramifié ayant chacun jusqu'à 5 atomes de carbone, ou par un groupe phényle, un groupe phénoxy ou un groupe thiophényle qui peuvent eux-mêmes porter un substituant fluoro, chloro, bromo, trifluorométhyle ou trifluorométhoxy,
ou bien les cycles sont éventuellement substitués par un groupe de formule -NR⁹R¹⁰,
dans laquelle
R⁹ et R¹⁰ sont identiques ou différents et ont la définition indiquée ci-dessus pour R³ et R⁴,
X est un reste alkylène ou un reste alcénylène linéaire ou ramifié ayant chacun jusqu'à 6 atomes de carbone, qui sont éventuellement substitués jusqu'à 2 fois par un groupe hydroxy,
R⁷ représente l'hydrogène, le fluor ou le chlore,
et
R⁸ est l'hydrogène, le fluor, le chlore, le brome, un groupe azido, trifluorométhyle, hydroxy, trifluorométhoxy, un groupe alkoxy linéaire ou ramifié ayant jusqu'à 4 atomes de carbone ou un reste de formule -NR¹¹R¹²,
dans laquelle
R¹¹ et R¹² sont identiques ou différents et ont la définition indiquée ci-dessus pour R³ et R⁴,
ou bien
R⁷ et R⁸ forment un groupe carbonyle conjointement avec l'atome de carbone,
E est un reste cyclopropyle, cyclobutyle, cyclopentyle, cyclohexyle ou cycloheptyle, ou bien
un reste alkyle linéaire ou ramifié ayant jusqu'à 6 atomes de carbone, qui porte éventuellement un substituant cyclopropyle, cyclobutyle, cyclopentyle, cyclohexyle, cycloheptyle ou un substituant hydroxy,
R¹ et R² forment ensemble une chaîne alkylénique ayant jusqu'à 5 atomes de carbone, qui est interrompue par un atome d'oxygène ou de soufre ou par le groupe -SO₂- ou un groupe -NR¹³, où
R¹³ représente l'hydrogène ou un reste alkyle ou acyle linéaire ou ramifié ayant dans chaque cas jusqu'à 5 atomes de carbone ou désigne un reste benzyle ou un reste phényle qui sont éventuellement substitués jusqu'à 2 fois identiques ou différentes par du fluor, du chlore, du brome, un groupe hydroxy, nitro, trifluprométhyle ou par un groupe alkyle ou un groupe acyle linéaire ou ramifié ayant dans chaque cas jusqu'à 4 atomes de carbone,
et le noyau hétérocyclique ainsi formé, qui peut aussi être condensé au benzène et peut contenir une double liaison, doit constamment être substitué par un reste de formule 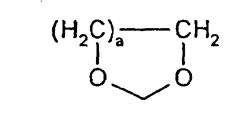 ou -OR¹⁴,
dans laquelle
a désigne le nombre 1, 2 ou 3
et
R¹⁴ représente l'hydrogène ou un reste alkyle, alkyle à substituant hydroxy ou alkoxycarbonyle linéaire ou ramifié ayant dans chaque cas jusqu'à 5 atomes de carbone, ou un reste de formule -SiR¹⁵R¹⁶R¹⁷,
dans laquelle
R¹⁵, R¹⁶ et R¹⁷ sont identiques ou différents et désignent un groupe phényle, un groupe alkyle linéaire ou ramifié ayant jusqu'à 5 atomes de carbone,
et/ou porte, le cas échéant, jusqu'à 4 substituants géminés, identiques ou différents, cyclopropyle, cyclopropyloxy, cyclopentyle, cyclopentyloxy, cyclohexyle, cyclohexyloxy, ou bien alkyle linéaire ou ramifié ayant jusqu'à 4 atomes de carbone, qui peut lui-même être substitué par un groupe hydroxy, trifluorométhyle, phényle ou par un groupe alkoxy linéaire ou ramifié ayant jusqu'à 3 atomes de carbone, et/ou est éventuellement substitué par un reste à liaison spiro, de formule 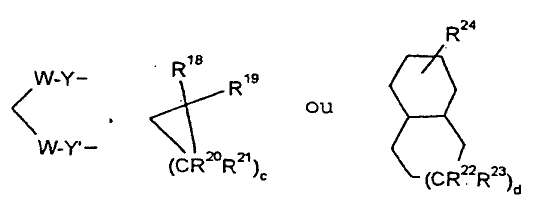 dans laquelle
W désigne un atome d'oxygène ou un atome de soufre,
Y et Y' forment ensemble une chaîne alkylénique linéaire ou ramifiée de 2 à 5 chaînons,
c désigne le nombre 1, 2, 3, 4, 5 ou 6,
d est le nombre 1 ou 2,
R¹⁸, R¹⁹, R²⁰, R²¹, R²², R²³ et R²⁴ sont identiques ou différents et représentent l'hydrogène, le reste trifluorométhyle, phényle, le fluor, le chlore, le brome ou un reste alkyle ou alkoxy linéaire ou ramifié ayant chacun jusqu'à 5 atomes de carbone, ou bien
R¹⁸ et R¹⁹ ou R²⁰ et R²¹ forment ensemble dans chaque cas une chaîne alkylénique linéaire ou ramifiée ayant jusqu'à 5 atomes de carbone, ou bien
R¹⁸ et R¹⁹ ou R²⁰ et R²¹ forment ensemble dans chaque cas un reste de formule 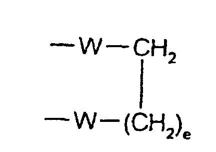
dans laquelle
W a la définition indiquée ci-dessus,
e est le nombre 1, 2, 3, 4, 5 ou 6,
et leurs sels et N-oxydes.

3. Pyridines condensées à un hétérocycle, de formule suivant la revendication 1
dans laquelle
A est un reste phényle qui est éventuellement substitué par du fluor, du chloré, du brome, un groupe hydroxy, trifluorométhyle, trifluorométhoxy, nitro ou par un groupe alkyle ou alkoxy linéaire ou ramifié ayant dans chaque cas jusqu'à 3 atomes de carbone ou,
D est un reste de formule
R⁵―X―
ou 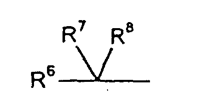 dans laquelle
R⁵ et R⁶ représentent, indépendamment l'un de l'autre, un reste cyclopropyle, naphtyle, phényle, pyridyle, quinolyle, indolyle, benzothiazolyle ou tétrahydronaphtalényle dont chacun est éventuellement substitué jusqu'à 2 fois identiques ou différentes par un groupe trifluorométhyle, trifluorométhoxy, le fluor, le chlore, le brome, un groupe hydroxy, carboxyle, amino, par un groupe alkyle, acyle, alkoxy ou alkoxycarbonyle linéaire ou ramifié ayant chacun jusqu'à 4 atomes de carbone ou par un groupe phényle, un groupe phénoxy ou un groupe thiophényle qui peuvent eux-mêmes être substitué par du fluor, du chlore, du brome, un radical trifluorométhyle ou trifluorométhoxy,
X est un groupe alkyle ou un reste alcényle linéaire ou ramifié ayant chacun jusqu'à 4 atomes de carbone et portant éventuellement un substituant hydroxy,
R⁷ est l'hydrogène ou le fluor,
et
R⁸ est l'hydrogène, le fluor, le chlore, le brome, un groupe azido, trifluorométhyle, hydroxy, amino, trifluorométhoxy ou méthoxy,
ou bien
R⁷ et R⁸ forment un groupe carbonyle conjointement avec l'atome de carbone,
E est un reste cyclopropyle, cyclobutyle, cyclopentyle, cyclohexyle ou cycloheptyle, ou bien
un reste alkyle linéaire ou ramifié ayant jusqu'à 5 atomes de carbone, qui porte éventuellement un substituant cyclopentyle ou cyclohexyle,
R¹ et R² forment ensemble une chaîne alkylénique ayant jusqu'à 4 atomes de carbone, qui est interrompue par un atome d'oxygène ou de soufre ou par le groupe -SO₂- ou un groupe -NR¹³,
où
R¹³ représente l'hydrogène ou un groupe alkyle ou acyle linéaire ou ramifié ayant dans chaque cas jusqu'à 4 atomes de carbone, ou représente un groupe benzyle ou un groupe phényle, qui sont éventuellement substitués jusqu'à 2 fois identiques ou différentes par du fluor, du chlore, du brome, un groupe hydroxy, nitro, trifluorométhyle ou par un groupe alkyle ou un groupe acyle linéaire ou ramifié ayant chacun jusqu'à 3 atomes de carbone,
et le noyau hétérocyclique ainsi formé, qui peut aussi être condensé au benzène et qui peut contenir une double liaison, doit constamment être substitué par un reste de formule 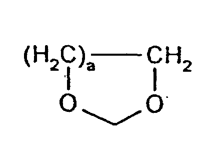 ou -OR¹⁴,
où
a désigne le nombre 1, 2 ou 3
et
R¹⁴ est l'hydrogène ou un groupe alkyle, alkyle à substituant hydroxy ou alkoxycarbonyle linéaire ou ramifié ayant chacun jusqu'à 4 atomes de carbone, ou un reste de formule -SiR¹⁵R¹⁶R¹⁷,
dans laquelle
R¹⁵, R¹⁶ et R¹⁷ sont identiques ou différents et désignent le groupe phényle, un groupe alkyle linéaire ou ramifié ayant jusqu'à 4 atomes de carbone,
et le noyau (R¹/R²) hétérocyclique et/ou condensé au benzène porte, le cas échéant, jusqu'à 3 substituants identiques ou différents, éventuellement géminés, alkoxy ou alkoxycarbonyle linéaire ou ramifié ayant chacun jusqu'à 3 atomes de carbone, hydroxy, carboxyle, ou bien phényle qui peut lui-même être substitué par du fluor, du chlore, du brome, un groupe trifluorométhyle ou par un groupe alkyle linéaire ou ramifié ayant dans chaque cas jusqu'à 3 atomes de carbone,
et/ou porte éventuellement jusqu'à 3 substituants identiques ou différents, éventuellement géminés, cyclopropyle, cyclopropyloxy, cyclopentyle, cyclopentyloxy, cyclohexyle, cyclohexyloxy, ou bien alkyle linéaire ou ramifié ayant jusqu'à 3 atomes de carbone, qui peut lui-même porter un substituant hydroxy, trifluorométhyle, phényle ou méthoxy,
et/ou est éventuellement substitué par un reste à liaison spiro, de formule 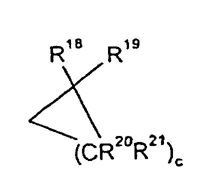 dans laquelle
c désigne le nombre 1, 2, 3, 4 ou 5,
R¹⁸, R¹⁹, R²⁰ et R²¹ sont identiques ou différents et représentent l'hydrogène, un groupe trifluorométhyle, phényle, le fluor, le chlore, le brome ou un groupe alkyle ou alkoxy linéaire ou ramifié ayant chacun jusqu'à 4 atomes de carbone, ou bien
R¹⁸ et R¹⁹ ou R²⁰ et R²¹ forment ensemble dans chaque cas une chaîne alkylénique linéaire ou ramifiée ayant jusqu'à 4 atomes de carbone,
ou bien
R¹⁸ et R¹⁹ ou R²⁰ et R²¹ forment ensemble dans chaque cas un reste de formule 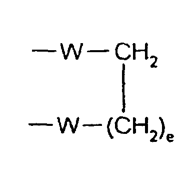
dans laquelle
W désigne un atome d'oxygène ou de soufre et
e désigne le nombre 1, 2, 3, 4 ou 5,
et leurs sels et N-oxydes.

4. Pyridines condensées à un hétérocycle, de formule suivant la revendication 1
dans laquelle
A désigne un groupe phényle qui est éventuellement substitué par du fluor, du chlore ou du brome,
et
E est un groupe cyclopentyle ou isopropyle.

5. Pyridines condensées à un hétérocycle suivant les revendications 1 à 4, utilisées comme médicament.

6. Procédé de production de pyridines condensées à un hétérocycle suivant les revendications 1 à 4, **caractérisé en ce qu'**on introduit tout d'abord le substituant D dont les composés de formule générale (II) 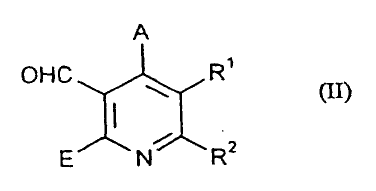 dans laquelle
A, E, R¹ et R² ont la définition indiquée ci-dessus,
avec des réactifs organométalliques au sens d'une réaction de Grignard ou de Wittig dans des solvants inertes,
et on fait varier ou on introduit, le cas échéant, les substituants indiqués pour A, E et/ou R¹ et R² selon des modes opératoires classiques.

7. Médicament contenant au moins une pyridine condensée à un hétérocycle suivant les revendications 1 à 4 ainsi qu'un auxiliaire de formulation acceptable du point de vue physiologique.

8. Médicament suivant la revendication 7, destiné au traitement de l'hyperlipoprotéinémie et de l'artériosclérose.

9. Utilisation de pyridines condensées à un hétérocycle suivant les revendications 1 à 4 pour la préparation de médicaments.

10. Utilisation suivant la revendication 9 pour la préparation de médicaments destinés au traitement de l'artériosclérose.
